# EUROPÄISCHE PATENTSCHRIFT

(11) **EP 0 820 437 B1**
(45) Veröffentlichungstag und Bekanntmachung des Hinweises auf die Patenterteilung: **25.07.2001**
(21) Anmeldenummer: 96908136.3
(22) Anmeldetag: 25.03.1996
(51) Int. Cl.: C07C 251/86, A01N 37/50

(54) **HYDRAZONOESSIGSÄUREAMIDE UND IHRE VERWENDUNG ALS SCHÄDLINGSBEKÄMPFUNGSMITTEL**
HYDRAZONOACETIC ACID AMIDES AND THE USE THEREOF AS PESTICIDES
AMIDES D'ACIDE HYDRAZONOACETIQUE ET LEUR UTILISATION COMME PESTICIDES

(30) Priorität: 05.04.1995 DE 19512617
(43) Veröffentlichungstag der Anmeldung: 28.01.1998
(73) Patentinhaber: BAYER AG, 51368 Leverkusen (DE)
(72) Erfinder: SEITZ, Thomas, D-40764 Langenfeld (DE); STENZEL, Klaus, D-40595 Düsseldorf (DE)
(86) Internationale Anmeldenummer: EP9601308
(87) Internationale Veröffentlichungsnummer: WO9631464

(56) Entgegenhaltungen:
- DE-A- 2 232 449
- US-A- 5 461 049
- CHEMICAL ABSTRACTS, vol. 112, no. 8, 19.Februar 1990 Columbus, Ohio, US; abstract no. 57392, HANNA, M. A. ET AL: "Photoxidation retardants. Spectra-structure correlation of some p-iodophenylcarbamoylarylhydrazidic bromides and their derivatives" XP002007770 & ACTA POLYM. (1989), 40(9), 582-6,
- LIEBIGS ANN. CHEM. (1989), (12), 1271-4, XP002007767 MODERHACK, DIETRICH: "Ring cleavage of 1,2,3-triazolium ions with nucleophiles"
- CHEMICAL ABSTRACTS, vol. 110, no. 21, 22.Mai 1989 Columbus, Ohio, US; abstract no. 192697, EL-REEDY, A. M. ET AL: "Reactions with monothiomalonamides: synthesis of polysubstituted thiazoles" XP002007771 & J. PRAKT. CHEM. (1988), 330(4), 521-9,
- CHEMICAL ABSTRACTS, vol. 108, no. 9, 29.Februar 1988 Columbus, Ohio, US; abstract no. 75196, MOHAREB, R. M. ET AL: "Polyfunctionally substituted pyridines from cyanoacetamide and cyanoacetanilide" XP002007772 & ARCH. PHARM. (WEINHEIM, GER.) (1987), 320(7), 599-604,
- CHEMICAL ABSTRACTS, vol. 95, no. 23, 7.Dezember 1981 Columbus, Ohio, US; abstract no. 203515, DUBENKO, R. G. ET AL: "Study of a series of arylhydrazones of glyoxylic acid derivatives. XXIX. Arylamides of 2,4-dinitrophenyl(acetyl)thioacetic acid in a reaction with aryldiazonium salts" XP002007773 & ZH. ORG. KHIM. (1981), 17(8), 1704-10,
- CHEMICAL ABSTRACTS, vol. 93, no. 9, 1.September 1980 Columbus, Ohio, US; abstract no. 94934, MONGUZZI, R. ET AL: "Synthesis and configurations of some.alpha.-hydrazonophenylacetic acids" XP002007774 & FARMACO, ED. SCI. (1980), 35(5), 394-404,
- CHEMICAL ABSTRACTS, vol. 87, no. 21, 21.November 1977 Columbus, Ohio, US; abstract no. 162131, MASHEVSKAYA, M. S.: "Synthesis and antimicrobial activity of benzoylformic acid amide arylhydrazones" XP002007775 & KHIM.-FARM. ZH. (1977), 11(7), 35-7,
- CHEMICAL ABSTRACTS, vol. 80, no. 21, 27.Mai 1974 Columbus, Ohio, US; abstract no. 120185, MODERHACK, DIETRICH ET AL: "2-(N-Alkylhydroxylamino)acid amides as potential 2-oxo acid amides" XP002007776 & CHEM.-ZTG. (1974), 98(2), 110-11,
- J. HETEROCYCL. CHEM. (1971), 8(3), 473-5, XP002007768 POPP, FRANK D. ET AL: "Reaction of N-acetylisatin with amines"
- TETRAHEDRON (1971), 27(12), 2517-28, XP002007769 SHAWALI, A. S. A. S. ET AL: "Synthesis and reactions of phenylcarbamoylarylhydrazidic chlorides"
- CHEMICAL ABSTRACTS, vol. 123, no. 25, 18.Dezember 1995 Columbus, Ohio, US; abstract no. 339119, FROHBERG, PETRA ET AL: "Lipoxygenase inhibitors. IV: Synthesis and cyclization reactions of open-chain N1-aryl-substituted amidrazones" XP002007777 & ARCH. PHARM. (WEINHEIM, GER.) (1995), 328(6), 505-16,

## Beschreibung

Die Erfindung betrifft neue Hydrazonoessigsäureamide, ein Verfahren zu ihrer Herstellung und ihre Verwendung als Schädlingsbekämpfungsmittel.

Hanna et al. (Acta Polym. (1989), 40(9), 582-586, Chemical Abstracts Nr. 112, No. 8, 19. Februar 1999) beschreiben die Verbindung N-(4-Iodophenyl)-alpha-(phenylhydrazono)-1-piperidin-acetamid. D. Moderhack (Liebigs Ann. Chem. 1989, 1271-1274) beschreibt die Ringspaltung von 1,2,3-Triazolium-Ionen mit Nucleophilen. El.-Reedy (J. Prakt. Chem. 1988, 330 (4), 521-9) berichtet über die Synthese von N-(4-Diphenyl)-alpha-(phenylhydrazono)-2-thiazolacetamid. Mohareb et al. (Arch. Pharm. (Weinheim, Ger.) 1987, 320(7), 599-604) beschreiben die Darstellung von 6-Diamin-3,5-dicyan-N-phenyl-alpha-((phenylhydrazono)-2-pyridinacetamid. Dubenko et al. berichten über die Reaktion von Arylamiden von 2,4-Dinitrophenyl(acetyl)thioessigsäure mit Aryldiazoniumsalzen (Zh. Org. Khim. 1981, 17 (8), 1704-10). Monguzzi et al. (Farmaco, Ed. Sci. 1980, 35(5), 394-404 beschreiben die Synthese und Konfigurationen von alpha-Hydrazonophenylessigsäuren. Mashevskaya et al. schreiben die Synthese von Arylhydrazonderivaten (Khim.-Farm. Zh. 1977, 11 (7), 35-7). In DE-A-2232449 werden Azofarbstoffe beschrieben. Moderhack et al. beschreiben die Synthese von N-Cyc.ohexal-alpha-[(2,4-dinitrophenyl)hydrazono]-benzeneacetamid. Popp et al. (J. Heterocycl. Chem. (1971), 8(3), 473-5 beschreiben die Reaktion von N-Acetylisatin mit Aminen. Shawali et al. beschreiben die Sythese und Reaktionen von phenylcarbamoylarylhydrazidchloriden (Tetrahedron (1971), 27(12), 2517-28. In US-5,461,049 wird die Darstellung von Amid Tetrazol Acat Inhibitoren beschrieben.

Eine fungizide Wirkung dieser Verbindungen ist jedoch nicht bekannt.

Es wurden neue Verbindung der allgemeinen Formel (I) gefunden, 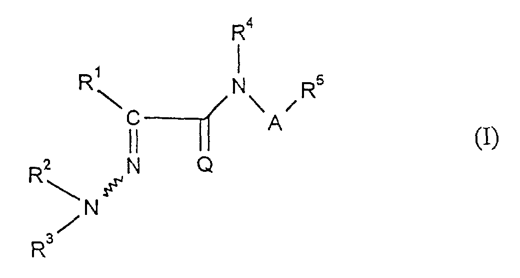 in welcher
- A: für gegebenenfalls substituiertes Alkylen steht,
- Q: für Sauerstoff oder Schwefel steht,
- R¹: für jeweils gegebenenfalls substituiertes Cycloalkyl, Cycloalkenyl, Aryl oder Heterocyclyl steht,
- R²: für Wasserstoff oder jeweils gegebenenfalls substituiertes Alkyl, Cycloalkyl, Cycloalkenyl, Aryl oder Heterocyclyl steht,
- R³, R⁴: gleich oder verschieden sind und jeweils für Wasserstoff oder jeweils gegebenenfalls substituiertes Alkyl oder Cycloalkyl stehen,
- R² und R³: zusammen mit dem Stickstoffatom, an das sie gebunden sind, einen gegebenenfalls substituierten Heterocyclylring bilden,
- R⁵: für jeweils gegebenenfalls substituiertes Cyloalkyl, Cycloalkenyl, Aryl oder Heterocyclyl steht.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Aryl steht für aromatische, mono oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte oder ungesättigte, sowie aromatische, ringförmige Verbindungen, in denen mindestens ein Ringglied ein Heteroatom, d. h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel.

Ringförmige Verbindungen bilden gegebenenfalls mit weiteren carbocyclischen oder heterocyclischen, ankondensierten oder überbrückten Ringen gemeinsam ein polycyclisches Ringsystem. Bevorzugt sind mono- oder bicyclische Ringsysteme, insbesondere mono- oder bicyclische, aromatische Ringsysteme.

Cycloalkyl steht für gesättigte, carbocyclische, ringförmige Verbindungen, die gegebenenfalls mit weiteren carbocyclischen, ankondensierten oder überbrückten Ringen ein polycyclisches Ringsystem bilden.

Cycloalkenyl steht für carbocyclische, ringförmige Verbindungen, die mindestens eine Doppelbindung enthalten und gegebenenfalls mit weiteren carbocyclischen, ankondensierten oder überbrückten Ringen ein polycyclisches Ringsystem bilden.

Schließlich wurde gefunden, daß die neuen Hydrazonoessigsäureamide der allgemeinen Formel (I) sehr starke fungizide Wirkung zeigen.

Die erfindungsgemäßen Verbindungen liegen gegebenenfalls als Mischungen verschiedener möglicher isomerer Formen, insbesondere von Stereoisomeren, wie z. B. E- und Z-, threo- und erythro-, sowie optischen Isomeren vor. Es werden sowohl die E- als auch die Z-Isomeren, wie auch die threo- und erythro-, sowie die optischen Isomeren sowie beliebige Mischungen dieser Isomeren beschrieben und beansprucht.

Gegenstand der Erfindung sind vorzugsweise Verbindungen der Formel (I), in welcher
- A: für gegebenenfalls einfach oder mehrfach, gleich oder verschieden substituiertes Alkylen mit 1 bis 6 Kohlenstoffatomen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl.
- Q: für Sauerstoff oder Schwefel steht,
- R¹: für jeweils gegebenenfalls einfach oder mehrfach, gleich oder verschieden substituiertes Aryl, Cycloalkyl oder Cycloalkenyl mit 3 bis 7 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
- R²: für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 4 Kohlenstoffatomen oder Cycloalkyl mit 3 bis 6 Kohlenstoffatomen oder für gegebenenfalls substituiertes Phenyl steht, wobei als Substituenten die in der vorstehenden Aufzählung für R¹ genannten ausgewählt sind,
- R² und R³: gemeinsam mit dem Stickstoffatom, an das sie gebunden sind, für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl, C₁-C₄-Alkyl (welche jeweils gegebenenfalls durch Halogen substituiert sein können) substituiertes Heterocyclyl stehen,
- R³, R⁴: gleich oder verschieden sind und jeweils für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 4 Kohlenstoffatomen oder Cycloalkyl mit 3 bis 6 Kohlenstoffatomen stehen,
- R⁵: für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I), in welcher
- A: für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methylpropylen) steht,
- Q: für Sauerstoff oder Schwefel steht,
- R¹: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy,
- R²: für Wasserstoff, Methyl, Ethyl, n- oder i-Propyl, n-, i-, s- oder t-Butyl oder für gegebenenfalls substituiertes Phenyl steht, wobei die Substituenten aus der für R¹ genannten vorstehenden Aufzählung ausgewählt sind,
- R² und R³: zusammen mit dem Stickstoffatom, an das sie gebunden sind, für einen gegebenenfalls durch Methyl, Ethyl, Methoxy oder Ethoxy substituierten Pyrrolidin-, Piperidin- oder Morpholinring stehen,
- R³, R⁴: gleich oder verschieden sind und jeweils für Wasserstoff oder für Methyl, Ethyl, n- oder i-Propyl, n-, i-, s- oder t-Butyl stehen,
- R⁵: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl , Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.
Besonders bevorzugt sind Verbindungen der Formel (I), in welcher
- A: für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methylpropylen) steht,
- Q: für Sauerstoff oder Schwefel steht,
- R¹: für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i- Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
- R²: für Wasserstoff, Methyl, Ethyl, n- oder i-Propyl oder für gegebenenfalls durch Methyl, Ethyl, Chlor, Fluor, Methoxy, Ethoxy oder Halogenmethoxy substituiertes Phenyl steht,
- R³: für Wasserstoff, Methyl, Ethyl, n- oder i-Propyl steht,
- R² und R³: zusammen mit dem Stickstoffatom, an das sie gebunden sind, für einen gegebenenfalls durch Methyl substituierten Pyrrolidin-, Piperidin- oder Morpholinring stehen,
- R⁴: für Wasserstoff, für Methyl oder für Ethyl steht,
- R⁵: für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I), in welcher
- A: für Methylen, 1,1-Ethylen, 1,2-Ethylen, 1,1-Propylen, 1,2-Propylen, 1,3-Propylen oder 2,2-Propylen steht,
- Q: für Sauerstoff steht,
- R¹: für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
- R²: für Wasserstoff, Methyl, Ethyl oder gegebenenfalls durch Methyl, Chlor, Methoxy oder Fluor substituiertes Methoxy substituiertes Phenyl steht,
- R³: für Wasserstoff, Methyl oder Ethyl steht,
- R² und R³: zusammen mit dem Stickstoffatom, an das sie gebunden sind, für einen Pyrrolidin-, Piperidin- oder Morpholinring stehen,
- R⁴: für Wasserstoff oder Methyl steht,
- R⁵: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Von ganz besonderem Interesse sind Verbindungen der Formel (I), in welcher der Rest R¹ für unsubstituiertes oder in 3- und/oder 4-Stellung substituiertes Phenyl, oder für unsubstituiertes oder in 4- und/oder 5-Stellung substituiertes Furanyl oder Thienyl steht, wobei als Substituenten die oben genannten und insbesondere Chlor, Brom, Fluor, Nitro, Methylsulfonyl, Phenyl, Phenoxy, Benzyloxy, Cyclopropyl, Cyclohexyl, Methyl, Ethyl, n-, i-Propyl, n-, i-, s-, t-Butyl, n-Pentyl, n-Hexyl, n-Heptyl, Methoxy, Ethoxy, Methylthio und Trifluormethyl ausgewählt sind oder jeweils gegebenenfalls durch Fluor substituiertes 3,4-Methylen- und Ethylendioxo, Propan-1,3-diyl und Butan-1,4-diyl substituiertes Phenyl oder Naphthyl, Benzofuranyl oder Benzothienyl bedeuten.

Insbesondere sind auch Verbindungen der Formel (I) bevorzugt, in denen R⁵ für in 3- und 4-Stellung durch Methoxy substituiertes Phenyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Bevorzugte Einzelverbindungen können den folgenden Tabellen entnommen werden:

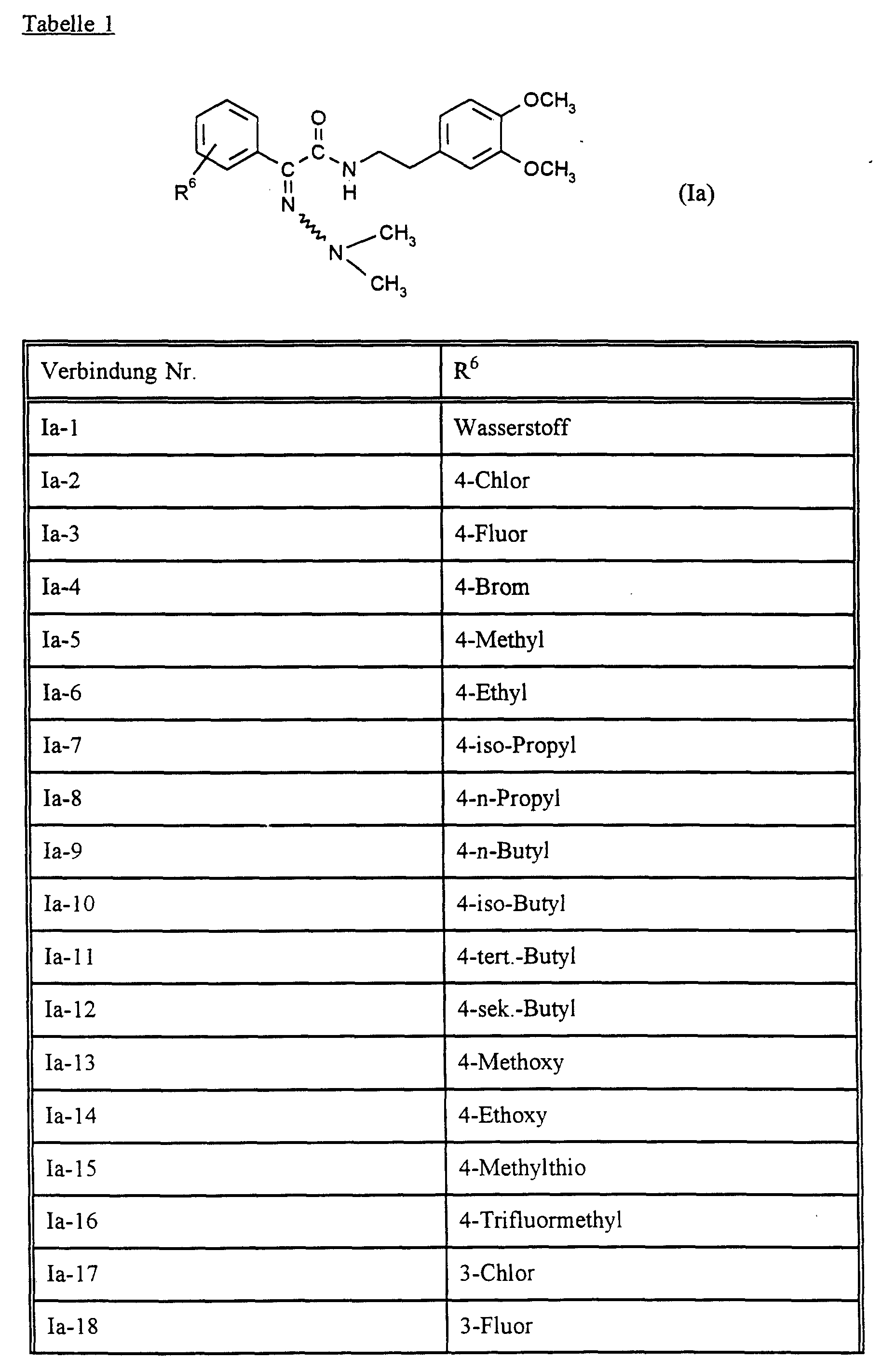

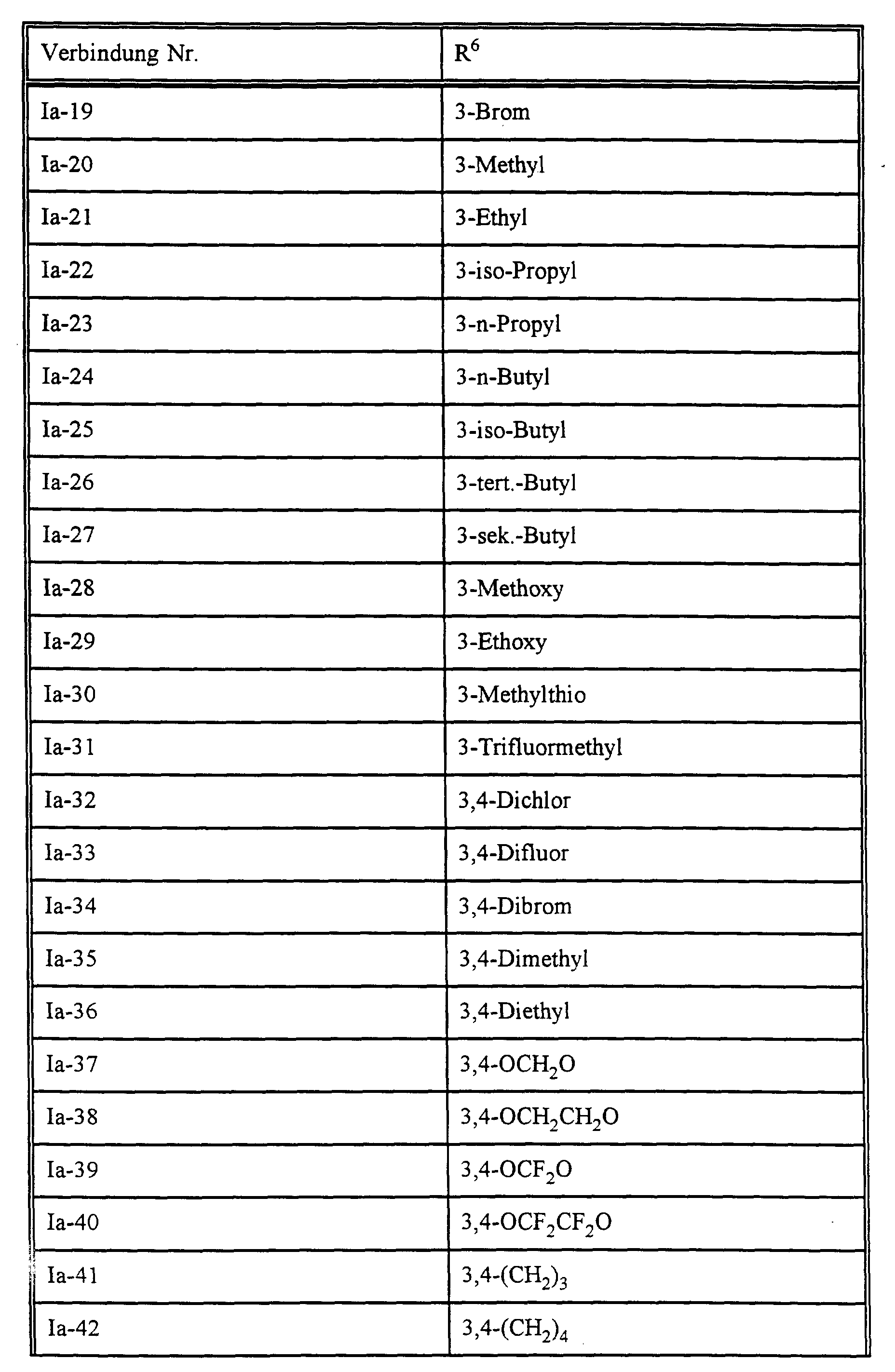

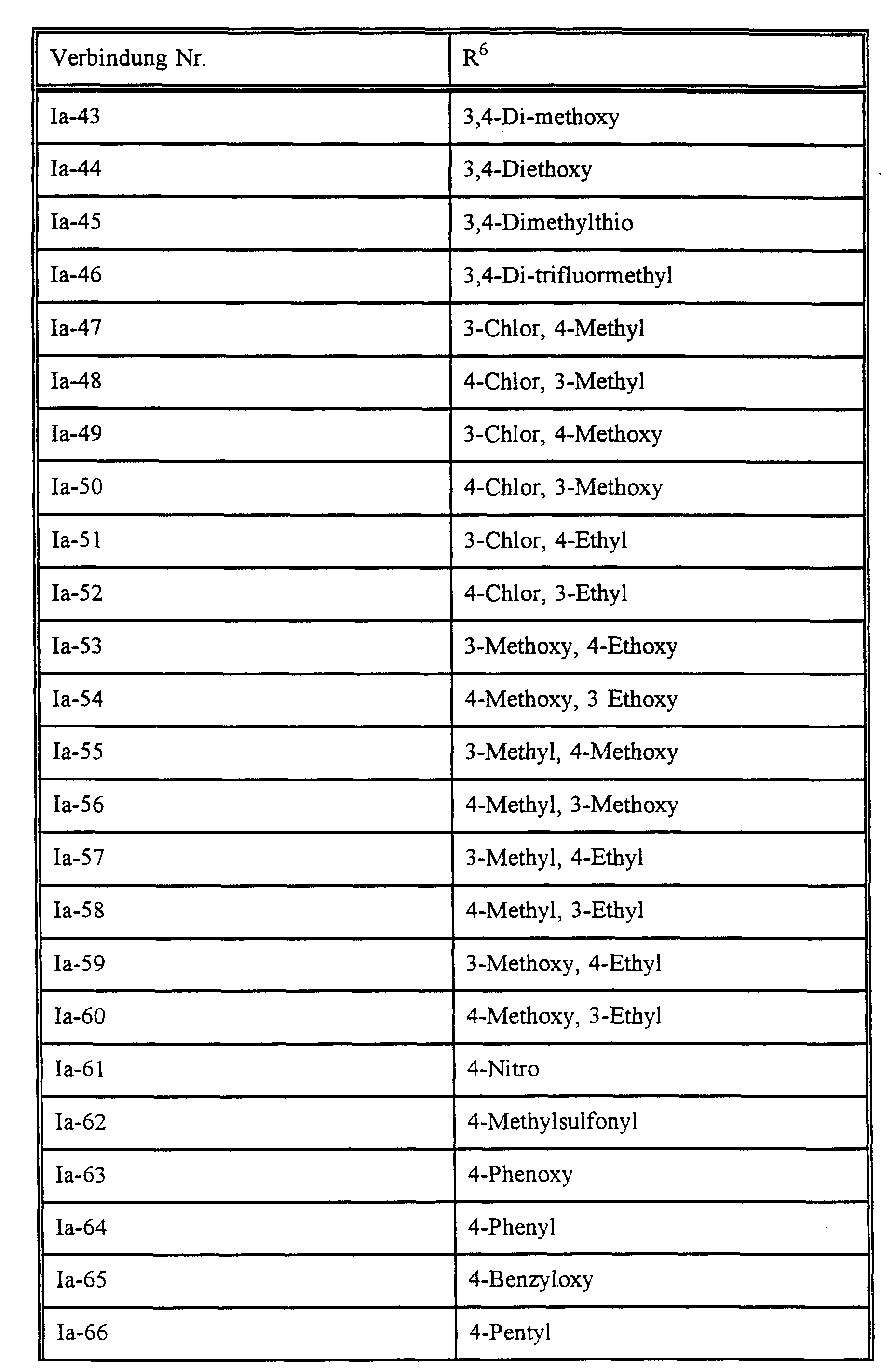

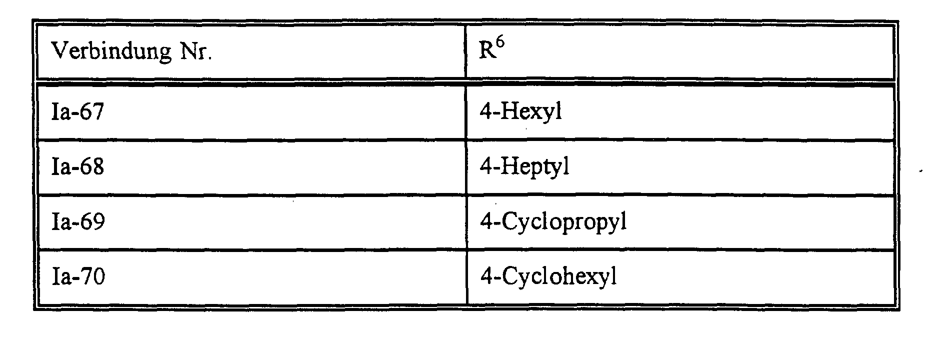

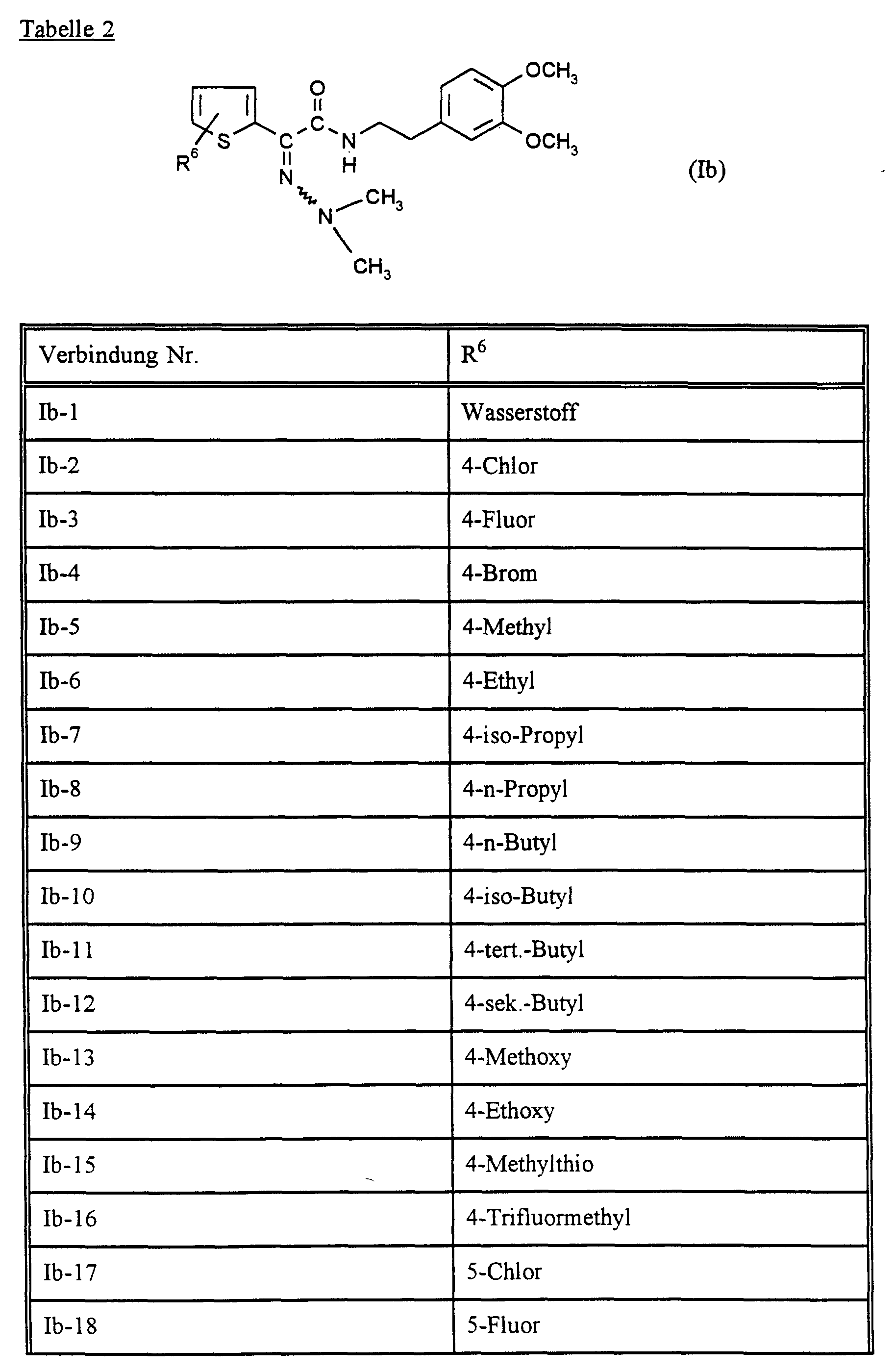

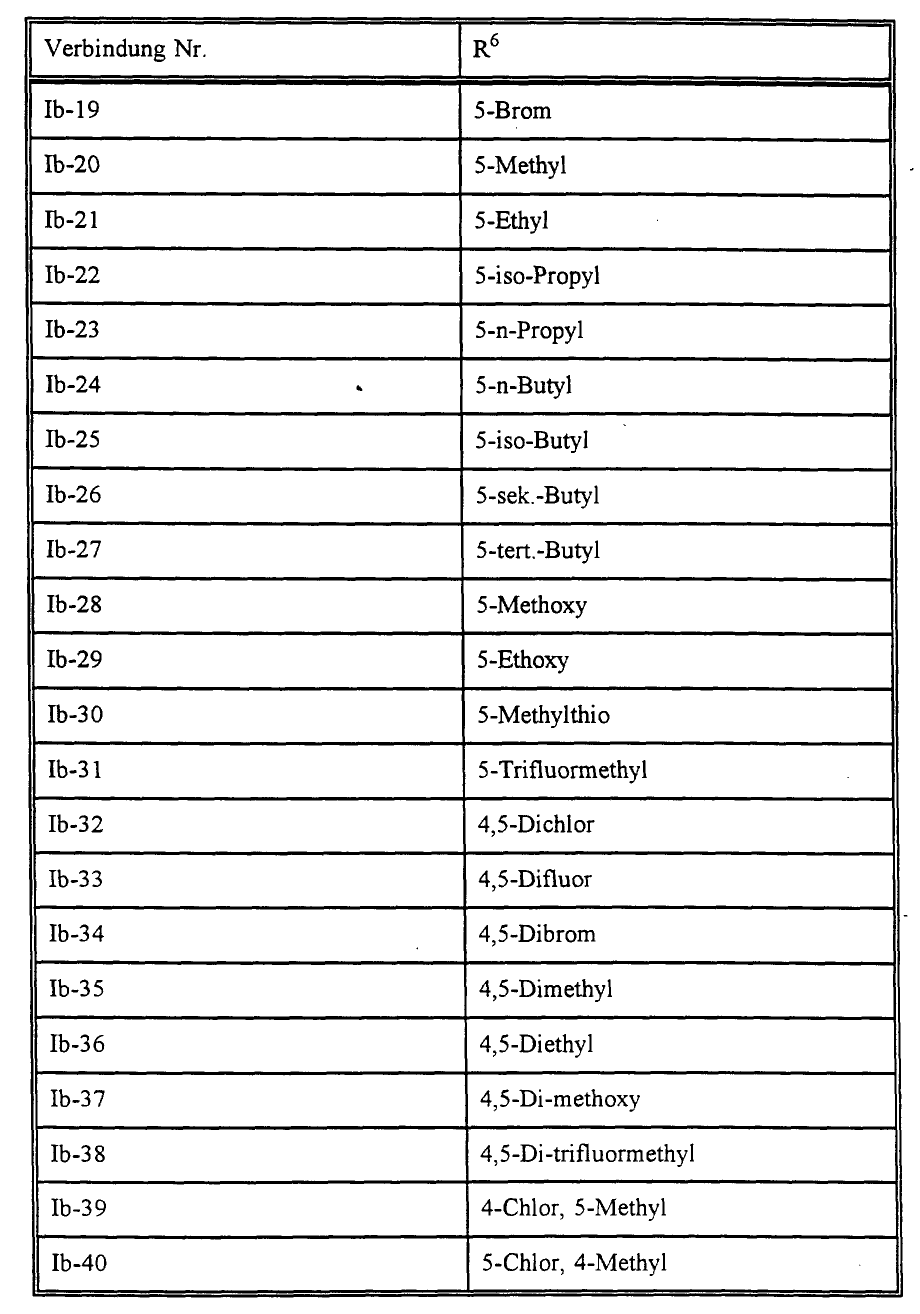

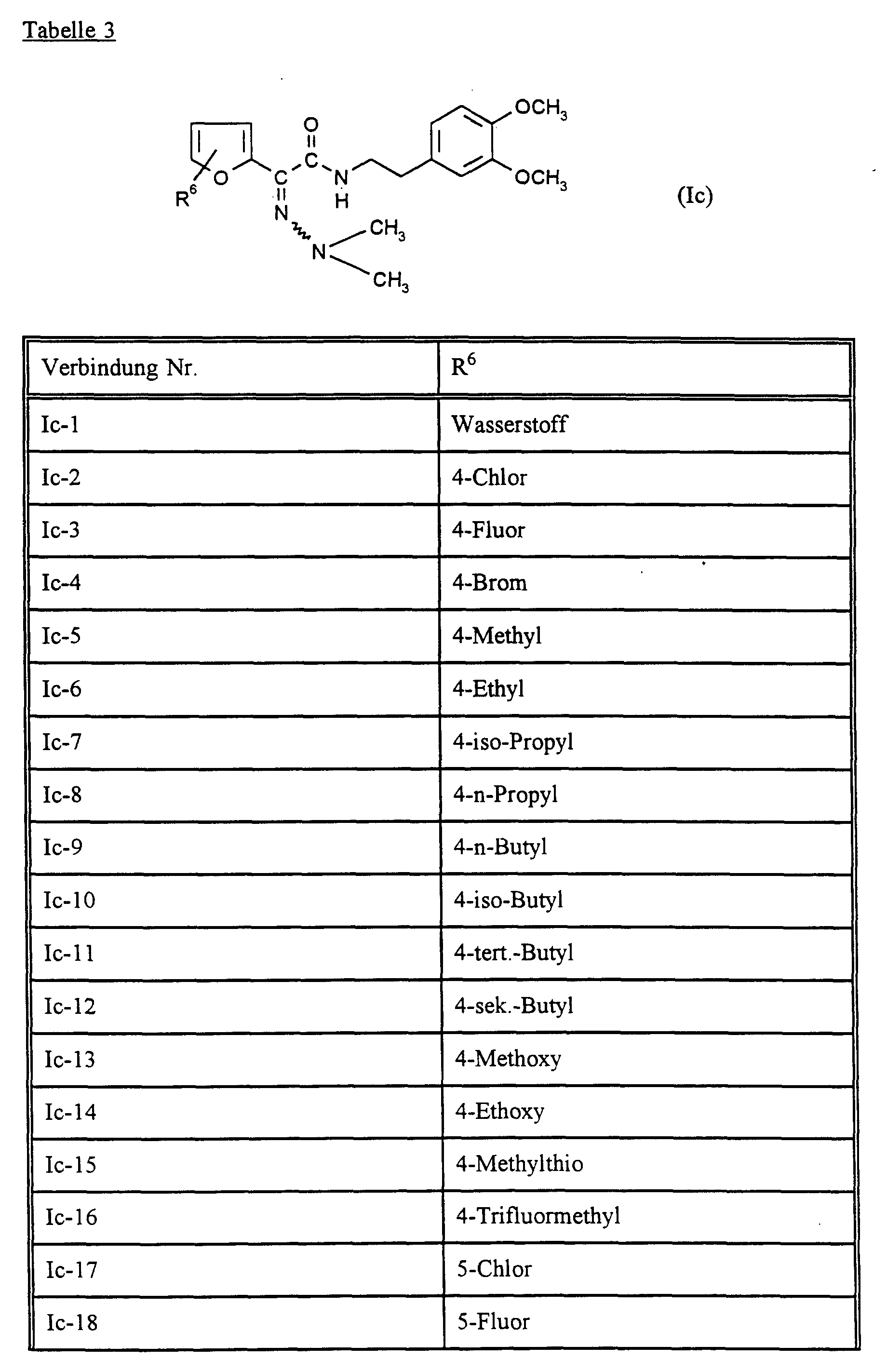

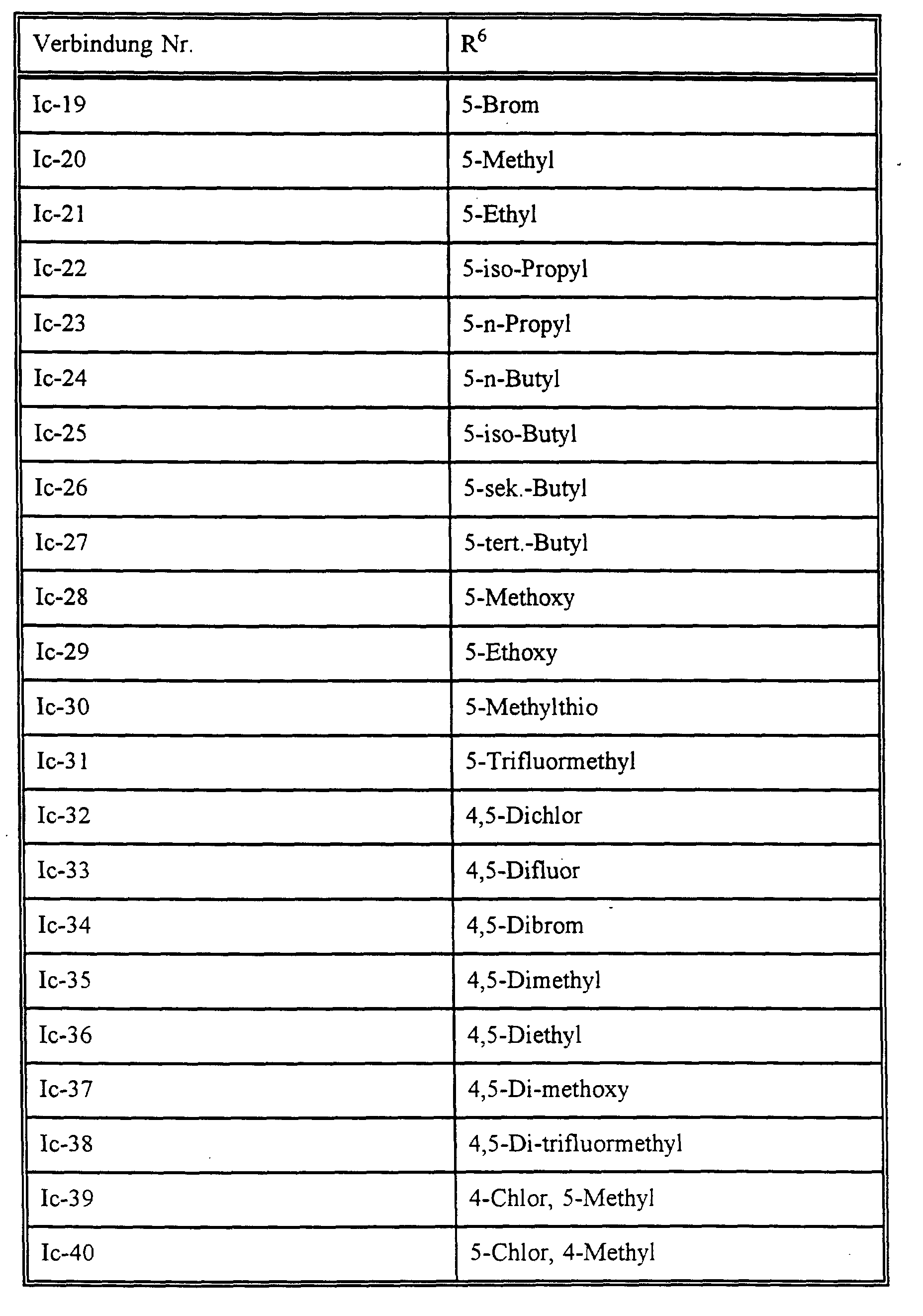

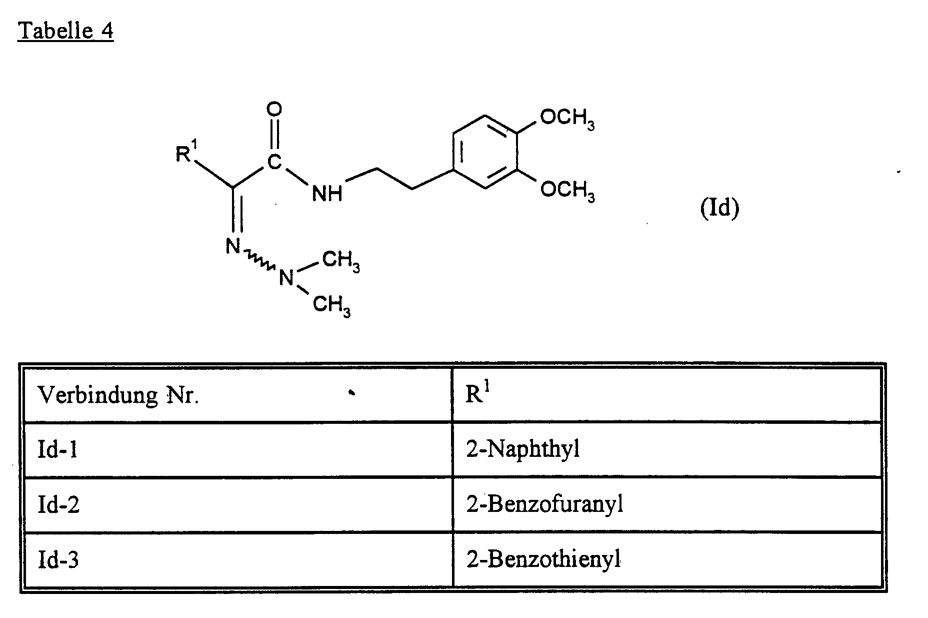

### Tabelle 5

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁵) ein Phenylrest steht, der die in den Verbindungen Ia-1 bis Ia-70 als R⁶ jeweils angegebenen Substituenten trägt.

### Tabelle 6

Verbindungen Ia-1 bis Id-3 entsprechend den Formeln Ia, Ib, Ic und Id, wobei an Stelle der 3,4-Dimethoxyphenyl-Gruppe (im allgemeinen bezeichnet als R⁵) einer der folgenden dreifach substituierten Phenylreste steht:
Substituenten: 3,4,5-Trimethoxy; 3,4,5-Trichlor; 3,4,5-Trimethyl.

Weiter wurde gefunden, daß man die neuen Hydrazonoessigsäureamide der allgemeinen Formel (I) erhält, wenn man Carbonsäurederivate der allgemeinen Formel (II) 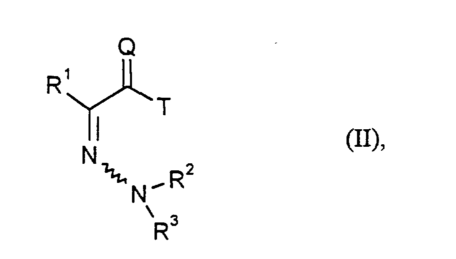 in welcher
- R¹, R², R³ und Q: die oben angegebene Bedeutung haben und
- T: für Hydroxy, Halogen oder Alkoxy steht,
mit einem Amin der allgemeinen Formel (III) 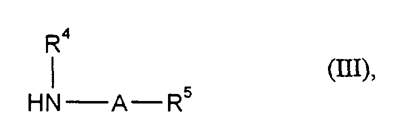 in welcher
- R⁴, R⁵: und A die oben angegebene Bedeutung haben,
- oder mit einem Hydrogenhalogenid hiervon -
gegebenenfalls in Gegenwart eines Säureakzeptors, gegebenenfalls in Gegenwart eines Kondensationsmittels und gegebenenfalls in Gegenwart eines Verdünnungsmittels umsetzt.

Die zur Durchführung des erfindungsgemäßen Verfahrens als Ausgangsstoffe benötigten Carbonsäurederivate sind durch die Formel (II) allgemein definiert. In dieser Formel (II) haben (Q, R¹, R² und R³) vorzugsweise insbesondere diejenige Bedeutungen, die bereits im Zusammenhang mit der Beschreibung der erfindungsgemäßen Verbindungen der Formel (I) als bevorzugt bzw. als insbesondere bevorzugt für (Q, R¹, R² und R³) angegeben wurden; T steht vorzugsweise für Alkoxy mit 1 bis 4 Kohlenstoffatomen, insbesondere für Methoxy oder Ethoxy, für Hydroxy oder Chlor.

Die Ausgangsstoffe der Formel (II) sind bekannt und/oder werden nach an sich bekannten Verfahren hergestellt (vgl. J. Heterocycl. Chem. (1990), 27(3), 487-95), Farmaco, Ed. Sci. (1980), 35(5), 394-404, Justus Liebigs Ann. Chem. (1969), 722, 38-44, Justus Liebigs Ann. Chem. (1969), 722, 29-37).

Die weiter als Ausgangsstoffe zu verwendeten Amine sind durch die Formel (III) allgemein definiert.

Die Ausgangsstoffe der Formel (III) sind bekannte organische Synthesechemikalien und/oder können nach an sich bekannten Verfahren hergestellt werden.

Das erfindungsgemäße Verfahren wird gegebenenfalls in Gegenwart eines geeigneten Säureakzeptors durchgeführt. Als solche kommen alle üblichen anorganischen oder organischen Basen infrage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren wird gegebenenfalls in Gegenwart eines Verdünnungsmittels durchgeführt. Als Verdünnungsmittel zur Durchführung des erfindungsgemäßen Verfahrens kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykol-dimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen -20°C und +200°C, vorzugsweise bei Temperaturen zwischen 0°C und 150°C.

Zur Durchführung des erfindungsgemäßen Verfahrens setzt man je Mol an Carbonsäurederivat der Formel (II) im allgemeinen 1 bis 5 Mol, vorzugsweise 1,0 bis 2,5 Mol an Amin ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens in einem größeren Bereich variiert werden. Im allgemeinen arbeitet man bei Temperaturen zwischen -78°C und +120°C, vorzugsweise bei Temperaturen zwischen -60°C und +25°C.

Zur Durchführung des erfindungsgemäßen Verfahrens zur Herstellung der Verbindungen der Formel (I) setzt man pro Mol des Hydrazonoessigsäurederivates der Formel (II) im allgemeinen 0,5 bis 5 Mol, vorzugsweise äquimolare Mengen an Amin der Formel (III) ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren kann auch als zweistufiger Prozess durchgeführt werden. Dabei werden die Hydrazonoessigsäurederivate der allgemeinen Formel (II) zunächst in eine aktivierte Form überführt und in einem anschließenden Schritt mit den Aminen der allgemeinen Formel (III) zu den erfindungsgemäßen Verbindungen der allgemeinen Formel (I) umgesetzt.

Als aktivierte Form der Hydrazonoessigsäurederivate der Formel (II) kommen alle Carboxy-aktivierten Derivate infrage, wie z.B. Säurehalogenide, bevorzugt Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Aminosäuren.

Die erfindungsgemäßen Wirkstoffe weisen eine starke mikrobizide Wirkung auf und werden zur Bekämpfung von unerwünschten Mikroorganismen praktisch eingesetzt. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus:
   Puccinia-Arten, wie beispielsweise Puccinia recondita;
   Tilletia-Arten, wie beispielsweise Tilletia caries;
   Ustilago-Arten, wie beispielsweise Ustilago nuda oder Ustilago avenae;
   Pellicularia-Arten, wie beispielsweise Pellicularia sasakii;
   Pyricularia-Arten, wie beispielsweise Pyricularia oryzae;
   Fusarium-Arten, wie beispielsweise Fusarium culmorum;
   Botrytis-Arten, wie beispielsweise Botrytis cinerea;
   Septoria-Arten, wie beispielsweise Septoria nodorum;
   Leptosphaeria-Arten, wie beispielsweise Leptosphaeria nodorum;
   Cercospora-Arten, wie beispielsweise Cercospora canescens;
   Alternaria-Arten, wie beispielsweise Alternaria brassicae;
   Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara- und Phytophthora-Arten eingesetzt.

Die Wirkstoffe werden in Abhängigkeit von ihren jeweiligen physikalischen und/oder chemischen Eigenschaften gegebenenfalls in übliche Formulierungen übergeführt, wie z. B. Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es werden in den Formulierungen gegebenenfalls Haftmittel wie Carboxymethylcellulose, natürliche und synthetische, pulverige, körnige oder latexförmige Polymere verwendet, wie z. B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Gegebenenfalls werden Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink zugesetzt.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb,
Cymoxanil, Cyproconazole, Cyprofuram,
Dichlorophen, Diclobutrazol, Diclofluanid, Diclomezin, Dicloran, Diethofencarb,
Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropi-morph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157
419, CGA 184699, Chloethocarb, Chlorethoxyfos, Chloretoxyfos, Chlorfenvinphos,
Chlorfluazuron, Chlormephos, Chlorpyrifos, Chlorpyrifos M, Cis-Resmethrin,
Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb. Fenothiocarb,
Fenoxycarb, Fenpropathrin, Fenpyrad, Fenpyroximat, Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lambda-cyhalothrin, Lufenuron,
Malathion, Mecarbam, Mervinphos, Mesulfenphos, Metaldehyd, Methacrifos,
Methamidophos, Methidathion, Methiocarb, Methomyl, Metolcarb, Milbemectin,
Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet,
Phosphamdon, Phoxim, Pirimicarb, Pirimiphos M, ,Pirimiphos A, Profenofos,
Profenophos, Promecarb, Propaphos, Propoxur, Prothiofos, Prothiophos, Prothoat,
Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Gegebenenfalls werden die erfindungsgemäßen Wirkstoffe auch mit anderen bekannten Wirkstoffen, wie Herbiziden oder auch mit Düngemitteln und Wachstumsregulatoren gemischt.

Die Wirkstoffe werden als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet. Die Anwendung geschieht in üblicher Weise, z.B. durch Gießen, Verspritzen, Versprühen, Verstreuen, Verstäuben, Verschäumen, Bestreichen usw. Gegebenenfalls werden die Wirkstoffe nach dem Ultra-Low-Volume-Verfahren ausgebracht oder die Wirkstoffzubereitung oder der Wirkstoff selbst wird in den Boden injiziert. Gegebenenfalls wird auch das Saatgut der Pflanzen behandelt.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiel 1:

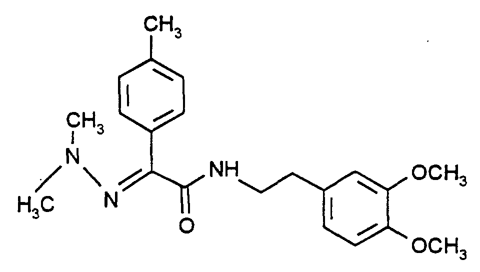

Es werden 0,8 g (3,4 mmol) 2-N,N-Dimethyl-hydrazono-2-(4-methylphenyl)-essigsäureethylester in 10 ml Methanol vorgelegt und nacheinander bei Raumtemperatur 0,6 g (3,4 mmol) 2-(3,4-Dimethoxyphenyl)-ethylamin und 1,2 g (6,8 mmol) 30 %ige methanolische Natriummethylat-Lösung dazugegeben.

Die Mischung wird 20 h bei 65°C gerührt, danach das Lösungsmittel abdestilliert, der Rückstand in Methylenchlorid aufgenommen, nacheinander mit Wasser, verdünnter Salzsäure und Wasser gewaschen und über Natriumsulfat getrocknet. Nach Abdestillieren des Lösungsmittels wird mit Petrolether/Essigester (10:1) an Kieselgel chromatographiert.

Man erhält 0,3 g (25 % d.Th.) N-[2-(3,4-Dimethoxyphenyl)-ethyl]-2-N,N-dimethylhydrazono-2-(4-methylphenyl)essigsäureamid. ¹H-NMR (CDCl₃, TMS): δ = 2,70 (S, 6H); 3,87 (S, 6H)

Analog Beispiel 1, sowie entsprechend den Angaben in der allgemeinen Verfahrensbeschreibung werden die in der nachstehenden Tabelle 7 genannten Verbindungen der Formel (Id) erhalten:

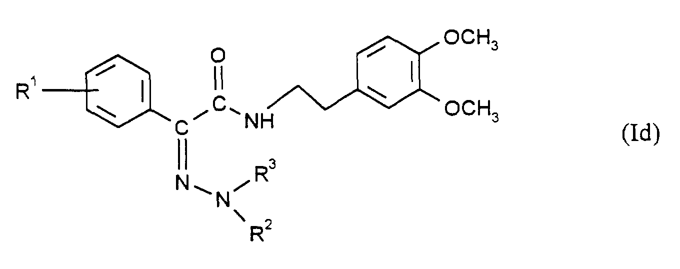

**Tabelle 7**

| Nr. | R¹ | R² | R³ | E/Z | phys. Konst. |
|---|---|---|---|---|---|
| 2 | 4-CH₃ | Ph | H | E/Z | log p: 3,44/4,22 |
| 3 | 4-CH₃ | 4-CF₃O-Ph | H | E/Z | log p: 4,16/4,92 |
| 4 | 4-CH₃ | CH₃ | CH₃ | Z | log p: 2,94 |
| 5 | 4-CH₃ | CH₃ | CH₃ | E | log p: 2,61 |
| 6 | 4-Br | CH₃ | CH₃ | E/Z | log p: 2,78/3,14 |
| 7 | 4-C₂H₅ | CH₃ | CH₃ | E/Z | log p: 2,89/3,22 |
| 8 | 4-CH₃ | 4-CF₃OPh | H | E | log p: 4,93 |
| 9 | 4-CH₃ | 4-CF₃OPh | H | Z | log p: 4,16 |
| 10 | 4-Br | Ph | H | E/Z | log p: 3,64/4,50 |
| 11 | 4-C₂H₅ | Ph | H | E/Z | log p: 3,81/4,61 |
| 12 | 4-C₂H₅ | CH₃ | H | E/Z | log p: 3,15/3,42 |
| 13 | 4-Cl | CH₃ | CH₃ | E/Z | log p: 2,66/3,04 |
| 14 | 4-Br | CH₃ | CH₃ | E | log p: 2,77 |
| 15 | 4-Br | CH₃ | CH₃ | Z | log p: 3,14 |
| 16 | 3,4-(CH₂)₄ | CH₃ | CH₃ | E/Z | log p: 2,84/3,19 |
| 17 | 3,4-(CH₂)₄ | CH₃ | H | E/Z | log p: 2,75/3,13 |
| 18 | 3,4-(CH₂)₄ | Ph | H | E/Z | log p: 4,16/5,01 |
| 19 | 4-CH₃ | Ph | H | Z | log p: 4,21 |
| 20 | 3,4-(CH₂)₃ | CH₃ | H | E/Z | log p: 3,19/3,47 |
| 21 | 3,4-(CH₂)₃ | CH₃ | CH₃ | E/Z | log p: 2,43/3,36 |
| 22 | 3,4-(CH₂)₃ | Ph | H | E/Z | log p: 3,89/4,72 |
| 23 | 4-Br | CH₃ | H | E/Z | log p: 2,88/3,42 |
| 24 | 4-Cl | Ph | H | E/Z | log p: 3,54/4,38 |
| 25 | 4-Cl | CH₃ | CH₃ | Z | log p: 3,03 |
| 26 | 4-C₂H₅ | CH₃ | CH₃ | Z | log p: 3,32 |
| 27 | 3,4-(CH₂)₃ | CH₃ | CH₃ | Z | log p: 3,36 |
| 28 | 4-Cl | Ph | H | Z | log p: 4,38 |
| 29 | 4-Cl | (CH₂)₅ | | E/Z | log p: 4,16/5,01 |
| 30 | 3,4-(CH₂)₄ | CH₃ | CH₃ | Z | log p: 3,19 |
| 31 | 3,4-(CH₂)₄ | CH₃ | H | Z | log p: 3,73 |
| 32 | 3,4-(CH₂)₃ | Ph | H | Z | log p: 4,72 |
| 33 | 4-CH₃ | (CH₂)₅ | | E/Z | log p: 3,66/3,84 |
| 34 | 4-Br | Ph | H | Z | log p: 4,48 |
| 35 | 4-Br | Ph | H | E | log p: 3,60 |
| 36 | 3,4-(CH₂)₄ | CH₃ | H | E | log p: 2,75 |
| 37 | 4-Br | (CH₂)₅ | | E/Z | log p: 3,56/4,06 |
| 38 | 4-C₂H₅ | (CH₂)₅ | | E/Z | log p: 3,11/4,25 |
| 39 | 3,4-(CH₂)₃ | (CH₂)₅ | | E/Z | log p: 3,05/4,31 |
| 40 | 3,4-(CH₂)₄ | (CH₂)₅ | | Z | log p: 4,65 |
| 41 | 4-Br | (CH₂)₂O(CH₂)₂ | | E/Z | log p: 2,72/2,91 |
| 42 | 4-Cl | (CH₂)₂O(CH₂)₂ | | E/Z | log p: 2,62/2,80 |
| 43 | 3,4-(CH₂)₄ | (CH₂)₂O(CH₂)₂ | | E/Z | log p: 3,25/3,36 |
| 44 | 3,4-(CH₂)₃ | (CH₂)₂O(CH₂)₂ | | E/Z | log p: 2,93/3,07 |
| 45 | 3,4-(CH₂)₃ | (CH₂)₄ | | E/Z | log p: 2,92/3,15 |
| 46 | 3,4-(CH₂)₄ | (CH₂)₄ | | E/Z | log p: 3,25/3,50 |
| 47 | 3,4-(CH₂)₄ | (CH₂)₂O(CH₂)₂ | | Z | log p: 3,36 |
| 48 | 3,4-(CH₂)₃ | (CH₂)₂O(CH₂)₂ | | Z | log p: 3,07 |

### Beispiel A

### Phytophthora-Test (Tomate) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

In diesem Test zeigt die erfindungsgemäße Verbindungen 1, 4, 14, 16, 17, 20, 29 und 31 bei einer Wirkstoffkonzentration von 100 ppm in der Spritzbrühe einen Wirkungsgrad von mehr als 80 %.

### Beispiel B

### Plasmopara-Test (Rebe) / protektiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 21°C und 90 % Luftfeuchtigkeit aufgestellt. Die Pflanzen werden dann angefeuchtet und 1 Tag in eine Feuchtekammer gestellt.

6 Tage nach der Inokulation erfolgt die Auswertung.

In diesem Test zeigt die erfindungsgemäßen Verbindungen 1, 4, 14, 16, 17, 20, 29 und 31 bei einer Wirkstoffkonzentration von 100 ppm in der Spritzbrühe einen Wirkungsgrad von mehr als 80 %.

## Patentansprüche

1. Verbindungen der Formel (I) 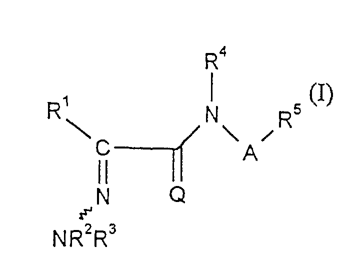 in welcher
A für gegebenenfalls substituiertes Alkylen steht,
Q für Sauerstoff oder Schwefel stehen,
R¹ für jeweils gegebenenfalls substituiertes Cycloalkyl, Cycloalkenyl, Aryl oder Heterocyclyl steht,
R² für Wasserstoff oder jeweils gegebenenfalls substituiertes Alkyl, Cycloalkyl, Cycloalkenyl, Aryl oder Heterocyclyl steht,
R³, R⁴ gleich oder verschieden sind und jeweils für Wasserstoff oder jeweils gegebenenfalls substituiertes Alkyl oder Cycloalkyl stehen,
R² und R³ zusammen mit dem Stickstoffatom, an das sie gebunden sind, einen gegebenenfalls substituierten Heterocyclylring bilden,
R⁵ für jeweils gegebenenfalls substituiertes Cyloalkyl, Cycloalkenyl, Aryl oder Heterocyclyl steht.

2. Verbindungen der Formel (I) gemäß Anspruch 1, in welcher
A für gegebenenfalls einfach oder mehrfach, gleich oder verschieden substituiertes Alkylen mit 1 bis 6 Kohlenstoffatomen steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl.
Q für Sauerstoff oder Schwefel steht,
R¹ für jeweils gegebenenfalls einfach oder mehrfach, gleich oder verschieden substituiertes Aryl, Cycloalkyl oder Cycloalkenyl mit 3 bis 7 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyaikylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
R² für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 4 Kohlenstoffatomen oder Cycloalkyl mit 3 bis 6 Kohlenstoffatomen oder für gegebenenfalls substituiertes Phenyl steht, wobei als Substituenten die in der vorstehenden Aufzählung für R¹ genannten ausgewählt sind,
R² und R³ gemeinsam mit dem Stickstoffatom, an das sie gebunden sind, für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl, C₁-C₄-Alkyl (welche jeweils gegebenenfalls durch Halogen substituiert sein können) substituiertes Heterocyclyl stehen,
R³, R⁴ gleich oder verschieden sind und jeweils für Wasserstoff oder für jeweils gegebenenfalls einfach oder mehrfach, gleich oder verschieden durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sein können) substituiertes Alkyl mit 1 bis 4 Kohlenstoffatomen oder Cycloalkyl mit 3 bis 6 Kohlenstoffatomen stehen,
R⁵ für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

3. Verbindungen der Foremel (I), gemäß Anspruch 1, in welcher
A für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i- Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy, Benzyloxy;
R² für Wasserstoff, Methyl, Ethyl, n- oder i-Propyl, n-, i-, s- oder t-Butyl oder für gegebenenfalls substituiertes Phenyl steht, wobei die Substituenten aus der für R¹ genannten vorstehenden Aufzählung ausgewählt sind,
R² und R³ zusammen mit dem Stickstoffatom, an das sie gebunden sind, für einen gegebenenfalls durch Methyl, Ethyl, Methoxy oder Ethoxy substituierten Pyrrolidin-, Piperidin- oder Morpholinring stehen,
R³, R⁴ gleich oder verschieden sind und jeweils für Wasserstoff oder für Methyl, Ethyl, n- oder i-Propyl, n-, i-, s- oder t-Butyl stehen,
R⁵ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

4. Verbindungen der Formel (I) gemäß Anspruch 1, in welcher
A für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten vorzugsweise aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
R² für Wasserstoff, Methyl, Ethyl, n- oder i-Propyl oder für gegebenenfalls durch Methyl, Ethyl, Chlor, Fluor, Methoxy, Ethoxy oder Halogenmethoxy substituiertes Phenyl steht,
R³ für Wasserstoff, Methyl, Ethyl, n- oder i-Propyl steht,
R² und R³ zusammen mit dem Stickstoffatom, an das sie gebunden sind, für einen gegebenenfalls durch Methyl substituierten Pyrrolidin-, Piperidin- oder Morpholinring stehen,
R⁴ für Wasserstoff, für Methyl oder für Ethyl steht,
R⁵ für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

5. Verbindungen der Formel (I), gemäß Anspruch 1, in welcher
A oder für Methylen, 1,1-Ethylen, 1,2-Ethylen, 1,1-Propylen, 1,2-Propylen, 1,3-Propylen oder 2,2-Propylen steht,
Q für Sauerstoff steht,
R¹ für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
R² für Wasserstoff, Methyl, Ethyl oder gegebenenfalls durch Methyl, Chlor, Methoxy oder Fluor substituiertes Methoxy substituiertes Phenyl steht,
R³ für Wasserstoff, Methyl oder Ethyl steht,
R² und R³ zusammen mit dem Stickstoffatom, an das sie gebunden sind, für einen Pyrrolidin-, Piperidin- oder Morpholinring stehen,
R⁴ für Wasserstoff oder Methyl steht,
R⁵ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i- Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

6. Schädlingsbekämpfungsmittel, gekennzeichnet durch einen Gehalt an mindestens einer Verbindung der Formel (I) nach Anspruch 1.

7. Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

8. Verwendung von Verbindungen der Formel (I) nach den Ansprüchen 1 bis 5 zur Bekämpfung von Schädlingen.

9. Verfahren zur Herstellung von Schädlingsbekämpfungsmitteln, dadurch gekennzeichnet, daß man Verbindungen der Formel (I) nach den Ansprüchen 1 bis 5 mit Streckmitteln und/oder oberflächenaktiven Mitteln vermischt.

10. Verfahren zur Herstellung von Verbindungen der Formel 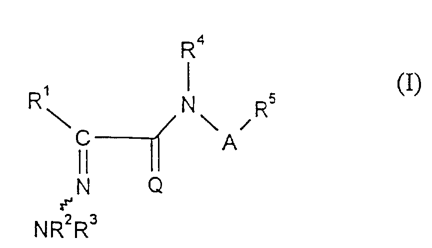 in welcher
A, Q, R¹, R², R³, R⁴ und R⁵ die in Anspruch 1 angegebenen Bedeutungen haben,
dadurch gekennzeichnet, daß man Carbonsäurederivate der allgemeinen Formel (II) 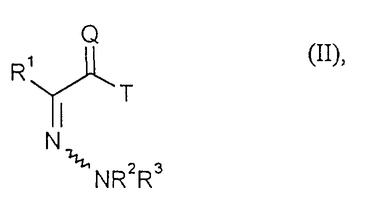 in welcher
R¹, R², R³ und Q die oben angegebene Bedeutung haben und
T für Hydroxy, Halogen oder Alkoxy steht,
mit einem Amin der allgemeinen Formel (III) 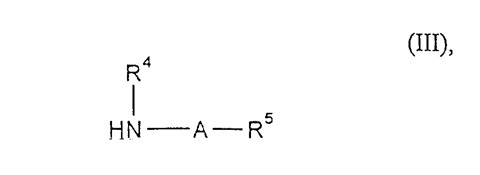 in welcher
R⁴, R⁵ und A die oben angegebene Bedeutung haben,
- oder mit einem Hydrogenhalogenid hiervon -
gegebenenfalls in Gegenwart eines Säureakzeptors, gegebenenfalls in Gegenwart eines Kondensationsmittels und gegebenenfalls in Gegenwart eines Verdünnungsmittels umsetzt.

## Claims

1. Compounds of the formula (I) 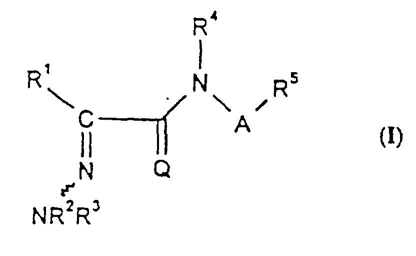 in which
A represents optionally substituted alkylene,
Q represents oxygen or sulfur,
R¹ represents in each case optionally substituted cycloalkyl, cycloalkenyl, aryl or heterocyclyl,
R² represents hydrogen or in each case optionally substituted alkyl, cycloalkyl, cycloalkenyl, aryl or heterocyclyl,
R³, R⁴ are identical or different and in each case represent hydrogen or in each case optionally substituted alkyl or cycloalkyl,
R² and R³, together with the nitrogen atom to which they are bonded, form an optionally substituted heterocyclyl ring,
R⁵ represents in each case optionally substituted cycloalkyl, cycloalkenyl, aryl or heterocyclyl.

2. Compounds of the formula (I) according to claim 1, in which
A represents alkylene having 1 to 6 carbon atoms which is optionally monosubstituted or polysubstituted by identical or different substituents, the possible substituents preferably being chosen from the following list:
halogen, cyano, nitro, amino, hydroxyl, formyl, carboxyl, carbamoyl, thiocarbamoyl;
in each case straight-chain or branched alkoxy, alkylthio, alkylsulfinyl or alkylsulfonyl having in each case 1 to 6 carbon atoms;
in each case straight-chain or branched alkenyl or alkenyloxy having in each case 2 to 6 carbon atoms;
in each case straight-chain or branched halogenoalkoxy, halogenoalkylthio, halogenoalkylsulfinyl or halogenoalkylsulfonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulfonyloxy, hydroximinoalkyl or
alkoximinoalkyl having in each case 1 to 6 carbon atoms in the individual alkyl parts;
cycloalkyl having 3 to 6 carbon atoms;
and aryl or heterocyclyl, in each case optionally monosubstituted or polysubstituted in an identical or different manner by
halogen, cyano and/or straight-chain or branched alkyl having 1 to 4 carbon atoms
and/or straight-chain or branched halogenoalkyl having 1 to 4 carbon atoms and 1 to 9 identical or different halogen atoms
and/or straight-chain or branched alkoxy or alkylthio having 1 to 4 carbon atoms
and/or straight-chain or branched halogenoalkoxy or halogenoalkylthio having 1 to 4 carbon atoms and 1 to 9 identical or different halogen atoms
and/or in each case divalent alkylene or dioxyalkylene having in each case 1 to 6 carbon atoms and optionally monosubstituted or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms,
Q represents oxygen or sulfur,
R¹ represents aryl, cycloalkyl or cycloalkenyl having 3 to 7 carbon atoms, or heterocyclyl having 3 to 12 ring members, in each case optionally monosubstituted or polysubstituted by identical or different substituents, the possible substituents preferably being chosen from the following list:
halogen, cyano, nitro, amino, hydroxyl, formyl, carboxyl, carbamoyl, thiocarbamoyl;
in each case straight-chain or branched alkyl, alkoxy, alkylthio, alkylsulfinyl or alkylsulfonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulfinyl or halogenoalkylsulfonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulfonyloxy, hydroximinoalkyl or
alkoximinoalkyl having in each case 1 to 8 carbon atoms in the individual alkyl parts;
in each case divalent alkylene or dioxyalkylene having in each case 1 to 6 carbon atoms and in each case optionally monosubstituted or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
and substituted aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, in each case optionally monosubstituted or polysubstituted in an identical or different manner by
halogen, cyano and/or straight-chain or branched alkyl having 1 to 4 carbon atoms
and/or straight-chain or branched halogenoalkyl having 1 to 4 carbon atoms and 1 to 9 identical or different halogen atoms
and/or straight-chain or branched alkoxy or alkylthio having 1 to 4 carbon atoms
and/or straight-chain or branched halogenoalkoxy or halogenoalkylthio having 1 to 4 carbon atoms and 1 to 9 identical or different halogen atoms
and/or in each case divalent alkylene or dioxyalkylene having in each case 1 to 6 carbon atoms and optionally monosubstituted or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms;
R² represents hydrogen, or represents alkyl having 1 to 4 carbon atoms or cycloalkyl having 3 to 6 carbon atoms, in each case optionally monosubstituted or polysubstituted in an identical or different manner by halogen, cyano, hydroxyl, amino, C₁₋C₄-alkoxy, C₁-C₄-alkylthio, C₁-C₄-alkylsulfinyl or C₁-C₄-alkylsulfonyl (which can in each case optionally be substituted by halogen), or represents optionally substituted phenyl, the substitutents chosen being those mentioned in the above list for R¹,
R² and R³, together with the nitrogen atom to which they are bonded, represent heterocyclyl, in each case optionally monosubstituted or polysubstituted in an identical or different manner by halogen, cyano, hydroxyl, amino, C₁-C₄-alkoxy, C₁-C₄-alkylthio, C₁-C₄-alkylsulfinyl or C₁-C₄-alkylsulfonyl, C₁-C₄-alkyl (which can in each case optionally be substituted by halogen),
R³, R⁴ are identical or different and in each case represent hydrogen, or represent alkyl having 1 to 4 carbon atoms or cycloalkyl having 3 to 6 carbon atoms, in each case optionally monosubstituted or polysubstituted in an identical or different manner by halogen, cyano, hydroxyl, amino, C₁-C₄-alkoxy, C₁-C₄-alkylthio, C₁-C₄-alkylsulfinyl or C₁-C₄-alkylsulfonyl (which can in each case be optionally substituted by halogen),
R⁵ represents aryl, cycloalkyl or cycloalkenyl having 3 to 8 carbon atoms, or heterocyclyl having 3 to 12 ring members, in each case optionally monosubstituted or polysubstituted by identical or different substituents, the possible substituents preferably being chosen from the following list:
halogen, cyano, nitro, amino, hydroxyl, formyl, carboxyl, carbamoyl, thiocarbamoyl;
in each case straight-chain or branched alkyl, alkoxy, alkylthio, alkylsulfinyl or alkylsulfonyl having in each case 1 to 6 carbon atoms;
in each case straight-chain or branched alkenyl or alkenyloxy having in each case 2 to 6 carbon atoms;
in each case straight-chain or branched halogenoalkyl, halogenoalkoxy, halogenoalkylthio, halogenoalkylsulfinyl or halogenoalkylsulfonyl having in each case 1 to 6 carbon atoms and 1 to 13 identical or different halogen atoms;
in each case straight-chain or branched halogenoalkenyl or halogenoalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulfonyloxy, hydroximinoalkyl or alkoximinoalkyl having in each case 1 to 6 carbon atoms in the individual alkyl parts;
in each case divalent alkylene or dioxyalkylene having in each case 1 to 6 carbon atoms and in each case optionally monosubstituted or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms;
cycloalkyl having 3 to 6 carbon atoms;
and aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, in each case optionally monosubstituted or polysubstituted in an identical or different manner by
halogen, cyano and/or straight-chain or branched alkyl having 1 to 4 carbon atoms
and/or straight-chain or branched halogenoalkyl having 1 to 4 carbon atoms and 1 to 9 identical or different halogen atoms
and/or straight-chain or branched alkoxy or alkylthio having 1 to 4 carbon atoms
and/or straight-chain or branched halogenoalkoxy or halogenoalkylthio having 1 to 4 carbon atoms and 1 to 9 identical or different halogen atoms
and/or in each case divalent alkylene or dioxyalkylene having in each case 1 to 6 carbon atoms and optionally monosubstituted or polysubstituted in an identical or different manner by halogen and/or straight-chain or branched alkyl having 1 to 4 carbon atoms and/or straight-chain or branched halogenoalkyl having 1 to 4 carbon atoms and 1 to 9 identical or different halogen atoms.

3. Compounds of the formula (I) according to claim 1, in which
A represents methylene, 1,1-ethylene, 1,2-ethylene, 1,1-, 1,2-, 1,3- or 2,2-proyplene, 1,1-, 1,2-, 1,3-, 1,4-, 2,2-, or 2,3-butylene or 1,1-, 1,2-or 1,3-(2-methyl-propylene), optionally monosubstituted or polysubstituted in an identical or different manner by fluorine, chlorine, cyano or methoxy,
Q represents oxygen or sulfur
R¹ represents in each case optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or 1-propyl, n-, i-, s- or t-butyl, n-pentyl, n-hexyl, n-heptyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl or trifluoromethylsulfonyl, methylamino, ethylamino, n- or i-propylamino, dimethylamino, diethylamino, acetyl, propionyl, acetyloxy, methoxycarbonyl, ethoxycarbonyl, methylsulfonyloxy, ethylsulfonyloxy, hydroiminomethyl, hydroximinoethyl, methoximinomethyl, ethoximinomethyl, methoximinoethyl or ethoximinoethyl;
in each case divalent trimethylene (propane-1,3-diyl), tetramethylene (butane-1,4-diyl),
methylenedioxy or ethylenedioxy, in each case optionally monosubstituted or polysubstituted in an identical or different manner by fluorine, chlorine, methyl, trifluoromethyl, ethyl or n- or i-propyl;
cyclopropyl, cyclobutyl, cyclopentyl or cyclohexyl;
phenyl, benzyl, phenoxy or benzyloxy, optionally substituted by the abovementioned substituents;
R² represents hydrogen, methyl, ethyl, n- or i-propyl or n-, i-, s- or t-butyl, or represents optionally substituted phenyl, the substituents being chosen from the list mentioned above for R¹,
R² and R³, together with the nitrogen atom to which they are bonded, represent a pyrrolidine, piperidine or morpholine ring optionally substituted by methyl, ethyl, methoxy or ethoxy,
R³, R⁴ are identical or different and in each case represent hydrogen, or represent methyl, methyl, n- or i-propyl or n-, i-, s- or t-butyl,
R⁵ represents in each case optionally mono- to trisubstituted cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i- propoxy, methylthio, ethylthio, n- or i- propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl or trifluoromethylsulfonyl, methylamino, ethylamino, n- or i-propyiamino, dimethylamino, diethylamino, acetyl, propionyl, acetyloxy, methoxycarbonyl, ethoxycarbonyl, methylsulfonyloxy, ethylsulfonyloxy, hydroximinomethyl, hydroiminoethyl, methoximinomethyl, ethoximinomethyl, methoximinoethyl or ethoximinoethyl,
in each case divalent trimethylene (propane-1,3-diyl), tetramethylene (butane-1,4-diyl), methylenedioxy or ethylenedioxy, in each case optionally monosubstituted or polysubstituted in an identical or different manner by fluorine, chlorine, methyl, trifluoromethyl, ethyl or n- or i-propyl,
cyclopropyl, cyclobutyl, cyclopentyl or cyclohexyl.

4. Compounds of the formula (I) according to claim 1, in which
A represents methylene, 1,1-ethylene, 1,2-ethylene, 1,1-, 1,2-, 1,3- or 2,2-propylene, 1,1-, 1,2-, 1,3-, 1,4-, 2,2- or 2,3-butylene or 1,1-, 1,2- or 1,3-(2-methyl-propylene), optionally monosubstituted or polysubstituted in an identical or different manner by fluorine, chlorine, cyano or methoxy,
Q represents oxygen or sulfur,
R¹ represents in each case optionally mono- to hexasubstituted cyclobutyl, cyclopentyl or cyclohexyl, preferred substituents being those mentioned below:
or represents in each optionally mono- to trisubstituted phenyl, naphthyl, furyl, benzofuranyl, thienyl, benzothienyl, pyridyl, quinolyl, tetrahydrofuryl, or perhydropyranyl, the possible substituents preferably being chosen from the following list:
fluorine, chlorine, bromine, cyano, nitro, methyl, ethyl, n- or i-propyl, n-, i-, s- or t- butyl, n-pentyl, n-hexyl, n-heptyl, methoxy, ethoxy, n- or i-propoxy, methylthio, ethylthio, n- or i-propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl, trifluoromethylsulfonyl, acetyl, propionyl, acetyloxy, methoxycarbonyl, ethoxycarbonyl, methylsulfonyloxy, ethylsulfonyloxy, hydroximinomethyl, hydroximinoethyl, methoximinomethyl, ethoximinomethyl, methoximinoethyl or ethoximinoethyl;
in each case divalent trimethylene (propane-1,3-diyl), tetramethylene (butane-1,4-diyl), methylenedioxy or ethylenedioxy, in each case optionally monosubstituted or polysubstituted in an identical or different manner by fluorine, chlorine, methyl, trifluoromethyl, ethyl or n- or i-propyl; cyclopropyl, cyclopentyl or cyclohexyl; phenyl, phenoxy or benzyloxy, optionally substituted by the abovementioned substituents;
R² represents hydrogen, methyl, ethyl or n- or i-propyl, or represents phenyl which is optionally substituted by methyl, ethyl, chlorine, fluorine, methoxy, ethoxy or halogenomethoxy,
R³ represents hydrogen, methyl, ethyl or n- or i-propyl,
R² and R³, together with the nitrogen atom to which they are bonded, represent a pyrrolidine, piperidine or morpholine ring, optionally substituted by methyl,
R⁴ represents hydrogen, or represents methyl, or represents ethyl,
R⁵ represents in each case optionally mono- to hexasubstituted cyclopropyl, cyclobutyl, cyclopentyl or cyclohexyl, preferred substituents being those mentioned below;
or represents in each case optionally mono- to trisubstituted phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i- propoxy, methylthio, ethylthio, n- or i- propylthio, methylsulfinyl, ethylsulfinyl, methylsulfonyl or ethylsulfonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl or trifluoromethylsulfonyl, methylamino, ethylamino, n- or i-propylamino, dimethylamino, diethylamino, acetyl, propionyl, acetyloxy, methoxycarbonyl, ethoxycarbonyl, methylsulfonyloxy, ethylsulfonyloxy, hydroximinomethyl, hydroximinoethyl, methoximinomethyl, ethoximinomethyl, methoximinoethyl or ethoximinoethyl,
in each case divalent trimethylene (propane-1,3-diyl), tetramethylene (butane-1,4-diyl), methylenedioxy or ethylenedioxy, in each case optionally monosubstituted or polysubstituted in an identical or different manner by fluorine, chlorine, methyl, trifluoromethyl, ethyl or n- or i-propyl,
cyclopropyl, cyclobutyl, cyclopentyl or cyclohexyl.

5. Compounds of the formula (I) according to claim 1, in which
A represents methylene, 1,1-ethylene, 1,2-ethylene, 1,1-propylene, 1,2-propylene, 1,3-propylene or 2,2-propylene,
Q represents oxygen,
R¹ represents phenyl, thienyl or furanyl, optionally mono- or disubstituted by bromine, chlorine, fluorine, nitro, methylsulfonyl, phenyl, phenyloxy, benzyloxy, cyclopropyl, cyclohexyl, methyl, ethyl, n- or i-propyl, n-, i-, s- or t- butyl, n-pentyl, n-hexyl, n-heptyl, methoxy, ethoxy, trifluoromethyl and/or methylthio, or represents phenyl substituted by 3,4-methylene- and ethylenedioxo, propane-1,3-diyl and butane-1,4-diyl, in each case optionally substituted by fluorine, or represents naphthyl, benzofuranyl or benzothienyl,
R² represents hydrogen, methyl, ethyl or phenyl which is optionally substituted by methyl, chlorine; methoxy or fluorine-substituted methoxy,
R³ represents hydrogen, methyl or ethyl,
R² and R³, together with the nitrogen atom to which they are bonded, represent a pyrrolidine, piperidine or morpholine ring,
R⁴ represents hydrogen or methyl,
R⁵ represents cyclohexyl or optionally mono- to trisubstituted phenyl, thienyl, furyl, benzofuryl, benzothienyl, pyridyl, pyrimidinyl, naphthyl or quinolyl, the possible substituents preferably being chosen from the following list:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i- propoxy, methylthio, ethylthio, n- or i- propylthio, methylsulfinyl, ethylsulfinyl, methylsulfonyl or ethylsulfonyl, trifluoromethyl, trifluoroethyl, difluoromethoxy, trifluoromethoxy, difluorochlormethoxy, trifluoroethoxy, difluoromethylthio, difluorochloromethylthio, trifluoromethylthio, trifluoromethylsulfinyl or trifluoromethylsulfonyl, methylamino, ethylamino, n- or i-propylamino, dimethylamino, diethylamino, acetyl, propionyl, acetyloxy, methoxycarbonyl, ethoxycarbonyl, methylsulfonyloxy,
ethylsulfonyloxy, hydroximinomethyl, hydroximinoethyl, methoximinomethyl, ethoximinomethyl, methoximinoethyl or ethoximinoethyl,
in each case divalent trimethylene (propane-1,3-diyl), tetramethylene (butane-1,4-diyl), methylenedioxy or ethylenedioxy, in each case optionally monosubstituted or polysubstituted in an identical or different manner by fluorine, chlorine, methyl, trifluoromethyl, ethyl or n- or i-propyl.

6. Compositions for combating pests, characterized by a content of at least one compound of the formula (I) according to claim 1.

7. Method of combating pests, characterized in that compounds of the formula (I) according to claim 1 are allowed to act on pests and/or their environment.

8. Use of compounds of the formula (I) according to claims 1 to 5 for combating pests.

9. Process for the preparation of compositions for combating pests, characterized in that compounds of the formula (I) according to claims 1 to 5 are mixed with extenders and/or surface-active agents.

10. Process for the preparation of compounds of the formula 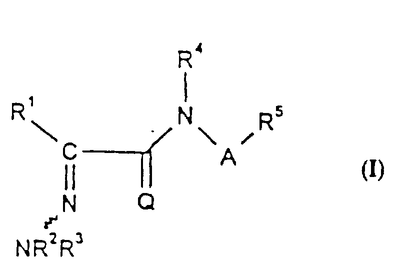 in which
A, Q, R¹, R², R³, R⁴ and R⁵ have the meanings given in claim 1,
characterized in that carboxylic acid derivatives of the general formula (II) 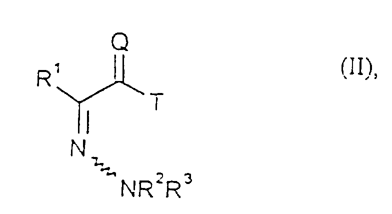 in which
R¹, R², R³ and Q have the abovementioned meaning and T represents hydroxyl, halogen or alkoxy,
are reacted with an amine of the general formula (III) 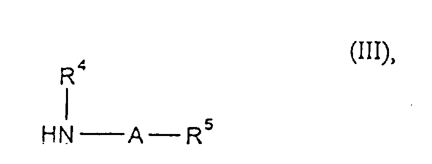 in which
R⁴, R⁵ and A have the abovementioned meaning,
or with a hydrogen halide thereof
optionally in the presence of an acid acceptor,
optionally in the presence of a condensing agent and
optionally in the presence of a diluent.

## Revendications

1. Composés de formule (I) 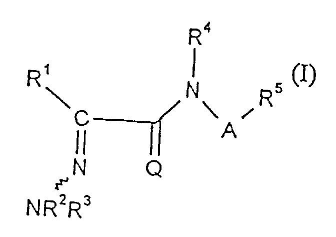 dans laquelle
A représente un groupe alkylène éventuellement substitué,
Q est l'oxygène ou le soufre,
R¹ représente un groupe cycloalkyle, cycloalcényle, aryle ou hétérocyclyle, chacun étant éventuellement substitué,
R² représente l'hydrogène ou un groupe alkyle, cycloalkyle, cycloalcényle, aryle ou hétérocyclyle, chacun étant éventuellement substitué,
R³, R⁴ sont égaux ou différents et représentent chacun l'hydrogène ou un groupe alkyle ou cycloalkyle, chacun étant éventuellement substitué,
R² et R³ forment conjointement avec l'atome d'azote auquel ils sont liés un noyau hétérocyclyle éventuellement substitué,
R⁵ est un groupe cycloalkyle, cycloalcényle, aryle ou hétérocyclyle, chacun étant éventuellement substitué.

2. Composés de formule (I) suivant la revendication 1, dans laquelle
A représente un groupe alkylène de 1 à 6 atomes de carbone éventuellement substitué une ou plusieurs fois identiques ou différentes, les substituants possibles étant de préférence choisis dans l'énumération ci-après :
halogène, cyano, nitro, amino, hydroxy, formyle, carboxy, carbamoyle, thiocarbamoyle ;
alkoxy, alkylthio, alkylsulfinyle ou alkylsulfonyle ayant chacun 1 à 6 atomes de carbone et chacun étant linéaire ou ramifié ;
alcényle ou alcényloxy ayant chacun 2 à 6 atomes de carbone et chacun étant linéaire ou ramifié ;
halogénalkoxy, halogénalkylthio, halogénalkylsulfinyle ou halogénalkylsulfonyle ayant chacun 1 à 6 atomes de carbone et 1 à 13 atomes d'halogènes identiques ou différents, chacun étant linéaire ou ramifié ;
halogénalcényle ou halogénalcényloxy ayant chacun 2 à 6 atomes de carbone et 1 à 13 atomes d'halogènes identiques ou différents, chacun étant linéaire ou ramifié ;
alkylamino, dialkylamino, alkylcarbonyle, alkylcarbonyloxy, alkoxycarbonyle, alkylsulfonyloxy, hydroximino-alkyle, ou alkoximino-alkyle ayant chacun 1 à 6 atomes de carbone dans les parties alkyle individuelles, chacun étant linéaire ou ramifié ;
cycloalkyle ayant 3 à 6 atomes de carbone ;
ainsi qu'aryle ou hétérocyclyle dont chacun est éventuellement substitué une ou plusieurs fois identiques ou différentes par
halogène, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, portant chacun éventuellement un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
Q représente l'oxygène ou le soufre,
R¹ est un groupe aryle, cycloalkyle ou cycloalcényle de 3 à 7 atomes de carbone ou hétérocyclyle à noyau de 3 à 12 chaînons, chacun portant le cas échéant un ou plusieurs substituants identiques ou différents, les substituants possibles étant choisis de préférence dans l'énumération suivante :
halogène, cyano, nitro, amino, hydroxy, formyle, carboxy, carbamoyle, thiocarbamoyie ;
alkyle, alkoxy, alkylthio, alkylsulfinyle ou alkylsulfonyle ayant chacun 1 à 6 atomes de carbone, chacun étant linéaire ou ramifié ;
alcényle ou alcényloxy ayant chacun 2 à 6 atomes de carbone, chacun étant linéaire ou ramifié ;
halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents, chacun étant linéaire ou ramifié ;
halogénalcényle ou halogénalcényloxy ayant chacun 2 à 6 atomes de carbone et 1 à 13 atomes d'halogènes identiques ou différents, chacun étant linéaire ou ramifié ;
alkylamino, dialkylamino, alkylcarbonyle, alkylcarbonyloxy, alkoxycarbonyle, alkylsulfonyloxy, hydroximino-alkyle, ou alkoximino-alkyle ayant chacun 1 à 8 atomes de carbone dans les parties alkyle individuelles, chacun étant linéaire ou ramifié ;
alkylène ou dioxyalkylène ayant chacun deux liaisons et chacun 1 à 6 atomes de carbone, chacun portant éventuellement un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio dont chacun est éventuellement substitué une ou plusieurs fois identiques ou différentes par :
halogène, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, dont chacun est éventuellement substitué une ou plusieurs fois identiques ou différentes par un halogène et/ou un reste alkyle linéaire ou ramifié ayant 1 à 4 atomes de carbone et/ou un reste halogénalkyle linéaire ou ramifié ayant 1 à 4 atomes de carbone et
1 à 9 atomes d'halogènes identiques ou différents,
R² représente l'hydrogène ou un groupe alkyle ayant 1 à 4 atomes de carbone ou cycloalkyle ayant 3 à 6 atomes de carbone, chacun étant éventuellement substitué une ou plusieurs fois identiques ou différentes par un halogène, un reste cyano, hydroxy, amino, alkoxy en C₁ à C₄, alkylthio en C₁ à C₄, alkylsulfinyle en C₁ à C₄ ou alkylsulfonyle en C₁ à C₄ (chacun pouvant être éventuellement substitué par un halogène) ou un groupe phényle éventuellement substitué, en choisissant comme substituants ceux qui sont mentionnés pour R¹ dans l'énumération précédente,
R² et R³ forment conjointement avec l'atome d'azote auquel ils sont liés un groupe hétérocyclyle portant éventuellement dans chaque cas un ou plusieurs substituants, identiques ou différents, halogéno, cyano, hydroxy, amino, alkoxy en C₁ à C₄, alkylthio en C₁ à C₄, alkylsulfinyle en C₁ à C₄ ou alkylsulfonyle en C₁ à C₄, alkyle en C₁ à C₄ (chacun pouvant être substitué le cas échéant par un halogène),
R³, R⁴ sont identiques ou différents et représentent chacun l'hydrogène ou un groupe alkyle ayant 1 à 4 atomes de carbone ou cycloalkyle ayant 3 à 6 atomes de carbone, chacun pouvant porter le cas échéant un ou plusieurs substituants, identiques ou différents, halogéno, cyano, hydroxy, amino, alkoxy en C₁ à C₄, alkylthio en C₁ à C₄, alkylsulfinyle en C₁ à C₄ ou alkylsulfonyle en C₁ à C₄ (chacun pouvant éventuellement être substitué par un halogène),
R⁵ représente un groupe aryle, cycloalkyle ou cycloalcényle de 3 à 8 atomes de carbone ou hétérocyclyle à noyau de 3 à 12 chaînons, chacun pouvant être substitué le cas échéant une ou plusieurs fois identiques ou différentes, les substituants possibles étant choisis de préférence dans l'énumération ci-après :
halogène, cyano, nitro, amino, hydroxy, formyle, carboxy, carbamoyle, thiocarbamoyie ;
alkyle, alkoxy, alkylthio, alkylsulfinyle ou alkylsulfonyle ayant chacun 1 à 6 atomes de carbone et chacun étant linéaire ou ramifié ;
alcényle ou alcényloxy ayant chacun 2 à 6 atomes de carbone et chacun étant linéaire ou ramifié ;
halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents, chacun étant linéaire ou ramifié ;
halogénalcényle ou halogénalcényloxy ayant chacun 2 à 6 atomes de carbone et 1 à 13 atomes d'halogènes identiques ou différents, chacun étant linéaire ou ramifié ;
alkylamino, dialkylamino, alkylcarbonyle, alkylcarbonyloxy, alkoxycarbonyle, alkylsulfonyloxy, hydroximino-alkyle ou alkoximino-alkyle ayant chacun 1 à 6 atomes de carbone dans les parties alkyle individuelles, chacun étant linéaire ou ramifié ;
alkylène ou dioxyalkylène ayant chacun deux liaisons et chacun 1 à 6 atomes de carbone, chacun portant le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy, ou hétérocyclylalkylthio, chacun étant éventuellement substitué ou une ou plusieurs fois identiques ou différentes par :
halogène, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, chacun étant éventuellement substitué une ou plusieurs fois identiques ou différentes par un halogène et/ou un reste alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents.

3. Composés de formule (I) suivant la revendication 1, dans laquelle
A est un groupe méthylène, 1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène) dont chacun est éventuellement substitué une ou plusieurs fois identiques ou différentes par du fluor, du chlore, un radical cyano ou méthoxy,
Q représente l'oxygène ou le soufre,
R¹ est un groupe cyclopropyle, cyclobutyle, cyclopentyle ou cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis de préférence dans l'énumération ci-après :
fluor, chlore, brome, cyano, nitro, amino, hydroxy, formyle, carboxy, carbamoyle, thiocarbamoyle, méthyle, éthyle, n-propyle, iso-propyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
triméthylène (propane-1,3-diyle), tétraméthylène (butane-1,4-diyle), méthylène-dioxy ou éthylène-dioxy ayant chacun deux liaisons, chacun étant éventuellement substitué une ou plusieurs fois identiques ou différentes par du fluor, du chlore, un radical méthyle, trifluorométhyle, éthyle, n-propyle ou isopropyle ;
cyclopropyle, cyclobutyle, cyclopentyle ou cyclohexyle ;
phényle, benzyle, phénoxy, benzyloxy portant éventuellement les substituants indiqués ci-dessus ;
R² représente l'hydrogène, un groupe méthyle, éthyle, n-propyle, isopropyle, n-butyle, isobutyle, sec.-butyle ou tertiobutyle ou un groupe phényle éventuellement substitué, les substituants étant choisis dans l'énumération donnée ci-dessus pour R¹ ;
R² et R³ forment conjointement avec l'atome d'azote auquel ils sont liés un noyau de pyrrolidine, pipéridine ou morpholine éventuellement substitué par un radical méthyle, éthyle, méthoxy ou éthoxy,
R³, R⁴ sont identiques ou différents et représentent chacun l'hydrogène ou un groupe méthyle, éthyle, n-propyle, isopropyle, n-butyle, isobutyle, sec.-butyle ou tertiobutyle,
R⁵ représente un groupe cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis de préférence dans l'énumération suivante :
fluor, chlore, brome, cyano, nitro, amino, hydroxy, formyle, carboxy, carbamoyle, thiocarbamoyle, méthyle, éthyle, n-propyle, iso-propyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
triméthylène (propane-1,3-diyle), tétraméthylène (butane-1,4-diyle), méthylène-dioxy ou éthylène-dioxy ayant chacun deux liaisons et chacun étant éventuellement substitué une ou plusieurs fois identiques ou différentes par du fluor, du chlore, un radical méthyle, trifluorométhyle, éthyle, n-propyle ou isopropyle ;
cyclopropyle, cyclobutyle, cyclopentyle ou cyclohexyle.

4. Composés de formule (I) suivant la revendication 1, dans laquelle
A représente un groupe méthylène, 1,1-éthylène, 1,2éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène) portant éventuellement un ou plusieurs substituants fluoro, chloro, cyano ou méthoxy identiques ou différents,
Q représente l'oxygène ou le soufre,
R¹ est un groupe cyclobutyle, cyclopentyle ou cyclohexyle dont chacun porte éventuellement 1 à 6 substituants, les substituants étant de préférence ceux qui sont mentionnés ci-dessous :
un groupe phényle, naphtyle, furyle, benzofurannyle, thiényle, benzothiényle, pyridyle, quinolyle, tétrahydrofuryle ou perhydropyrannyle portant éventuellement dans chaque cas 1 à 3 substituants, les substituants possibles étant choisis de préférence dans l'énumération ci-après :
fluor, chlore, brome, cyano, nitro, méthyle, éthyle, n-propyle, iso-propyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle ;
triméthylène (propane-1,3-diyle), tétraméthylène (butane-1,4-diyle), méthylène-dioxy ou éthylène-dioxy ayant chacun deux liaisons et portant chacun le cas échéant 1 à 3 substituants, identiques ou différents, fluor, chlore, méthyle, trifluorométhyle, éthyle, n-propyle ou isopropyle ; cyclopropyle, cyclopentyle, cyclohexyle ; phényle, phénoxy, benzyloxy éventuellement substitué par les substituants mentionnés ci-dessus ;
R² représente l'hydrogène, un groupe méthyle, éthyle, n-propyle, isopropyle ou un groupe phényle éventuellement substitué par un radical méthyle, éthyle, chloro, fluoro, méthoxy, éthoxy ou halogénométhoxy,
R³ représente l'hydrogène, un groupe méthyle, éthyle, n-propyle ou isopropyle,
R² et R³ forment conjointement avec l'atome d'azote auquel ils sont liés un noyau de pyrrolidine, pipéridine ou morpholine éventuellement substitué par un radical méthyle,
R⁴ représente l'hydrogène, un groupe méthyle ou un groupe éthyle,
R⁵ représente un groupe cyclopropyle, cyclobutyle, cyclopentyle ou cyclohexyle dont chacun est éventuellement substitué 1 à 6 fois, les substituants mentionnés ci-dessous étant préférés ;
un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle dont chacun porte éventuellement 1 à 3 substituants, les substituants possibles étant choisis de préférence dans l'énumération suivante :
fluor, chlore, brome, cyano, nitro, amino, hydroxy, formyle, carboxy, carbamoyle, thiocarbamoyle, méthyle, éthyle, n-propyle, iso-propyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n- propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
triméthylène (propane-1,3-diyle), tétraméthylène (butane-1,4-diyle), méthylène-dioxy ou éthylène-dioxy ayant chacun deux liaisons et chacun étant éventuellement substitué une ou plusieurs fois identiques ou différentes par du fluor, du chlore, un radical méthyle, trifluorométhyle, éthyle, n-propyle ou isopropyle ;
cyclopropyle, cyclobutyle, cyclopentyle ou cyclohexyle.

5. Composés de formule (I) suivant la revendication 1, dans laquelle
A est un groupe méthylène, 1,1-éthylène, 1,2-éthylène, 1,1-propylène, 1,2-propylène, 1,3- propylène ou 2,2-propylène,
Q représente l'oxygène,
R¹ est un groupe phényle, thiényle, furannyle portant éventuellement 1 ou 2 substituants bromo, chloro, fluoro, nitro, méthylsulfonyle, phényle, phényloxy, benzyloxy, cyclopropyle, cyclohexyle, méthyle, éthyle, n-propyle, isopropyle, n-butyle, isobutyle, sec.-butyle, tertiobutyle, n-pentyle, n-hexyle, n-heptyle, méthoxy, éthoxy, trifluorométhyle et/ou méthylthio ou un groupe phényle portant éventuellement un substituant 3,4-méthylène- et éthylène-dioxo, propane-1,3-diyle et butane-1,4-diyle dont chacun est éventuellement substitué par du fluor, ou un groupe naphtyle, benzofurannyle ou benzothiényle,
R² représente l'hydrogène, un groupe méthyle, éthyle ou un groupe phényle éventuellement substitué par un radical méthyle, chloro, méthoxy ou fluoro,
R³ représente l'hydrogène, un groupe méthyle ou éthyle,
R² et R³ forment, conjointement avec l'atome d'azote auquel ils sont liés, un noyau de pyrrolidine, pipéridine ou morpholine,
R⁴ représente l'hydrogène ou un groupe méthyle,
R⁵ représente un groupe cyclohexyle ou un groupe phényle, thiényle, furyle, benzofuryle, benzothiényle, pyridyle, pyrimidinyle, naphtyle, quinolyle portant éventuellement 1 à 3 substituants, les substituants possibles étant de préférence choisis dans l'énumération ci-après :
fluor, chlore, brome, cyano, nitro, amino, hydroxy, formyle, carboxy, carbamoyle, thiocarbamoyle, méthyle, éthyle, n-propyle, iso-propyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ;
triméthylène (propane-1,3-diyle), tétraméthylène (butane-1,4-diyle), méthylène-dioxy ou éthylène-dioxy ayant chacun deux liaisons, chacun étant éventuellement substitué une ou plusieurs fois identiques ou différentes par un radical fluoro, chloro, méthyle, trifluorométhyle, éthyle, n-propyle ou isopropyle ;

6. Compositions pesticides, caractérisées par une teneur en au moins un composé de formule (I) suivant la revendication 1.

7. Procédé pour combattre des parasites, caractérisé en ce qu'on fait agir des composés de formule (I) suivant la revendication 1 sur les parasites et/ou sur leur milieu.

8. Utilisation de composés de formule (I) suivant les revendications 1 à 5 pour combattre des parasites.

9. Procédé de préparation de compositions pesticides, caractérisé en ce qu'on mélange des composés de formule (I) suivant les revendications 1 à 5 avec des diluants et/ou des agents tensioactifs.

10. Procédé de production de composés de formule 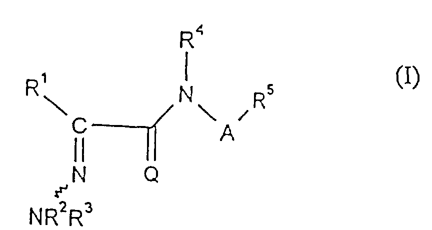 dans laquelle
A, Q, R¹, R², R³, R⁴ et R⁵ ont les définitions indiquées dans la revendication 1,
caractérisé en ce qu'on fait réagir des dérivés d'acides carboxyliques de formule générale (II) 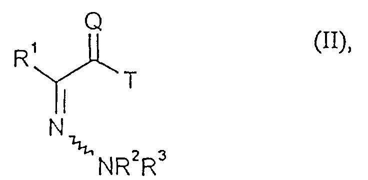 dans laquelle
R¹, R², R³ et Q ont la définition indiquée ci-dessus et
T représente un groupe hydroxy, un halogène ou un groupe alkoxy,
avec une amine de formule générale (III) 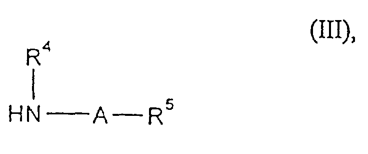 dans laquelle
R⁴, R⁵ et A ont la définition indiquée ci-dessus,
- ou avec un halogénhydrate de cette amine -
le cas échéant en présence d'un accepteur d'acide, éventuellement en présence d'un agent de condensation et en présence éventuelle d'un diluant.
